# EUROPEAN PATENT APPLICATION

(11) **EP 1 346 983 A2**
(43) Date of publication of application: **24.09.2003**
(21) Application number: 03076931.9
(22) Date of filing: 19.02.1999
(51) Int. Cl.: C07D 209/48, A61K 31/404, A61P 35/00, C07D 401/04, C07D 295/08

(54) **Phthalimido arylpiperazines as alpha 1A receptor antagonists useful in the treatment of benign prostatic hyperplasia**

(30) Priority: 20.02.1998 US 75510 P
(62) Divisional of application: 99908303.3
(71) Applicant: Ortho-McNeil Pharmaceutical, Inc., Raritan, NJ 08869-0602 (US)
(72) Inventor: Prouty, Catherine P., Doylestown, Pennsylvania 18901 (US); Murray, William V., Belle Mead, New Jersey 08502 (US); Kuo, Gee-Hong, Scotch Plains, New Jersey 07076 (US)
(74) Representative: Mercer, Christopher Paul

(57) **Abstract**

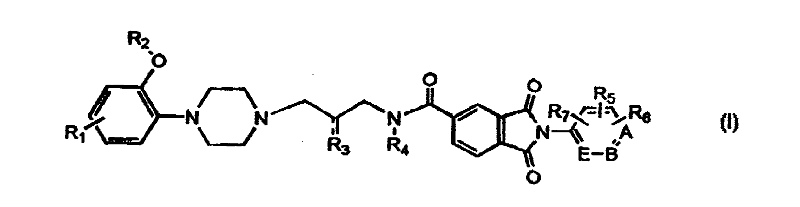

This invention relates to a series of heterocyclic substituted piperazines of Formula (I), pharmaceutical compositions containing them and intermediates used in their manufacture. The compounds of the invention selectively inhibit binding to the α-1ₐ adrenergic receptor, a receptor which has been implicated in benign prostatic hyperplasia. As such the compounds are potentially useful in the treatment of this disease.

## Description

### FIELD OF THE INVENTION

This invention relates to a series of phthalimido arylpiperazine derivatives, pharmaceutical compositions containing them as well as processes and intermediates used in their manufacture. The compounds of the invention selectively inhibit binding to the α1ₐ adrenergic receptor, a receptor which has been implicated in benign prostatic hyperplasia. As such the compounds are potentially useful in the treatment of this disease.

### BACKGROUND

Benign prostatic hyperplasia (BPH), a nonmalignant enlargement of the prostate, is the most common benign tumor in men. Approximately 50% of all men older than 65 years have some degree of BPH and a third of these men have clinical symptoms consistent with bladder outlet obstruction (Hieble and Caine, 1986). In the U.S., benign and malignant diseases of the prostate are responsible for more surgery than diseases of any other organ in men over the age of fifty.

There are two components of BPH, a static and a dynamic component. The static component is due to enlargement of the prostate gland, which may result in compression of the urethra and obstruction to the flow of urine from the bladder. The dynamic component is due to increased smooth muscle tone of the bladder neck and the prostate itself (which interferes with emptying of the bladder) and is regulated by alpha 1 adrenergic receptors (α1-ARs). The medical treatments available for BPH address these components to varying degrees, and the therapeutic choices are expanding.

Surgical treatment options address the static component of BPH and include transurethral resection of the prostate (TURP), transurethral incision of the prostate (TUIP), open prostatectomy, balloon dilatation, hyperthermia, stents and laser ablation. TURP is the preferred treatment for patients with BPH and approximately 320,000 TURPs were performed in the U.S. in 1990 at an estimated cost of $2.2 billion (Weis et al., 1993). Although an effective treatment for most men with symptomatic BPH, approximately 20 - 25% of patients do not have a satisfactory long-term outcome (Lepor and Rigaud, 1990). Complications include retrograde ejaculation (70-75% of patients), impotence (5-10%), postoperative urinary tract infection (5-10%), and some degree of urinary incontinence (2-4%) (Mebust et al., 1989). Furthermore, the rate of reoperation is approximately 15-20% in men evaluated for 10 years or longer (Wennberg et al., 1987).

Apart from surgical approaches, there are some drug therapies which address the static component of this condition. Finasteride (Proscar, Merck), is one such therapy which is indicated for the treatment of symptomatic BPH. This drug is a competitive inhibitor of the enzyme 5α-reductase which is responsible for the conversion of testosterone to dihydrotestosterone in the prostate gland (Gormley et al., 1992). Dihydrotestosterone appears to be the major mitogen for prostate growth, and agents which inhibit 5α-reductase reduce the size of the prostate and improve urine flow through the prostatic urethra. Although finasteride is a potent 5α-reductase inhibitor and causes a marked decrease in serum and tissue concentrations of dihydrotestosterone, it is only moderately effective in treating symptomatic BPH (Oesterling, 1995). The effects of finasteride take 6-12 months to become evident and for many men the clinical improvement is minimal (Barry, 1997).

The dynamic component of BPH has been addressed by the use of adrenergic receptor blocking agents (α1-AR blockers) which act by decreasing the smooth muscle tone within the prostate gland itself. A variety of α1-AR blockers (terazosin, prazosin, and doxazosin) have been investigated for the treatment of symptomatic bladder outlet obstruction due to BPH, with terazosin (Hytrin, Abbott) being the most extensively studied. Although the α1-AR blockers are well-tolerated, approximately 10-15% of patients develop a clinically adverse event (Lepor, 1995). The undesirable effects of all members of this class are similar, with postural hypotension being the most commonly experienced side effect (Lepor et al., 1992). In comparison to the 5α-reductase inhibitors, the α1-AR blocking agents have a more rapid onset of action (Steers, 1995). However, their therapeutic effect, as measured by improvement in the symptom score and the peak urinary flow rate, is moderate. (Oesterling, 1995)

The use of α1-AR antagonists in the treatment of BPH is related to their ability to decrease the tone of prostatic smooth muscle, leading to relief of the obstructive symptoms. Adrenergic receptors are found throughout the body play a dominant role in the control of blood pressure, nasal congestion, prostrate function and other processes (Harrison et al., 1991). However, there are a number of cloned α1-AR receptor subtypes: α1ₐ-AR, α1_{b}-AR and α1_{d}-AR (Bruno et al., 1991; Forray et al., 1994; Hirasawa et al., 1993; Ramarao et al., 1992; Schwinn et al., 1995; Weinberg et al., 1994). A number of labs have characterized the α1-ARs in human prostate by functional, radioligand binding, and molecular biological techniques (Forray et al., 1994; Hatano et al., 1994; Marshall et al., 1992; Marshall et al., 1995; Yamada et al., 1994). These studies provide evidence in support of the concept that the α1ₐ-AR subtype comprises the majority of α1-ARs in human prostatic smooth muscle and mediates contraction in this tissue. These findings suggest that the development of a subtype-selective α1ₐ-AR antagonist might result in a therapeutically effective agent with reduced side effects for the treatment of BPH.

### SUMMARY OF THE INVENTION

The compounds of this invention selectively bind to the α1ₐ-AR receptor, antagonize the activity of said receptor and are selective for prostate tissue over aortic tissue. As such, these represent a viable treatment for BHP without the side effects associated with known α1-AR antagonists.

The invention includes compounds of Formula I 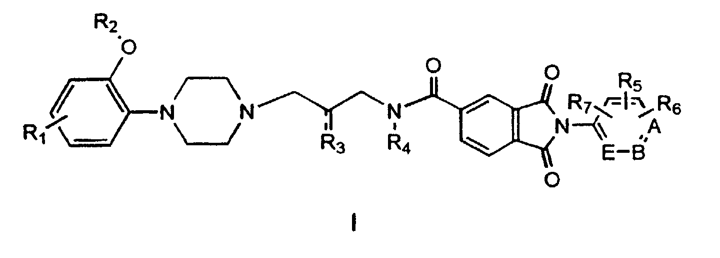 wherein:
- R₁: is hydrogen, halogen, C₁₋₅alkoxy, hydroxyl, or C₁₋₅alkyl;
- R₂: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₃: is hydrogen, hydroxy or C₁₋₅alkoxy if the hashed line is absent or
is oxygen if the hashed line is present;
- R₄: is hydrogen, C₁₋₅alkyl, phenylC₁₋₅alkyl or substituted phenylC₁₋₅alkyl
where the phenyl substitutents are independently selected from one or more members of the group consisting of C₁₋₅alkyl, C₁₋₅alkoxy, and trihaloC₁₋₅alkyl;
- R₅: is hydrogen, halogen, hydroxy, C₁₋₈alkyl, substituted C₁₋₈alkyl
where the alkyl substitutents are independently selected from one or more halogens,
C₁₋₅alkoxy, amino, C₁₋₅alkylamino, diC₁₋₅alkylamino, C₁₋₅alkylcarbonyl,
C₁₋₅alkoxycarbonyl, nitrile, or nitro;
- R₆: is hydrogen, halogen, hydroxy, C₁₋₈alkyl, substituted C₁₋₈alkyl
where the alkyl substitutents are independently selected from one or more halogens,
C₁₋₅alkoxy, amino, C₁₋₅alkylamino, diC₁₋₅alkylamino, C₁₋₅alkylcarbonyl,
C₁₋₅alkoxycarbonyl, or nitro;
- R₇: is hydrogen, halogen, hydroxy, C₁₋₈alkyl, substituted C₁₋₈alkyl
where the alkyl substitutents are independently selected from one or more halogens,
C₁₋₅alkoxy, amino, C₁₋₅alkylamino, diC₁₋₅alkylamino, C₁₋₅alkylcarbonyl,
C₁₋₅alkoxycarbonyl, or nitro;
- A: is nitrogen or carbon;
- B: is nitrogen or carbon;
- E: is nitrogen or carbon;
with the proviso that only one of A, B, or E is nitrogen;
pharmaceutically acceptable salts thereof; and
stereoisomers, racemic mixtures, as well as enantiomers thereof.

In addition this invention contemplates pharmaceutical compositions containing an effective dose of compounds of Formula I. Still further this invention contemplates methods of treating diseases associated with the α1ₐ adrenergic receptor consisting of administering an effective dose of a compound of Formula I to a mammal. This invention also contemplates a method of treating benign prostatic hyperplasia consisting of administering an effective dose of a compound of Formula I to a mammal.

Aside from compounds of Formula I, this invention contemplates intermediate compounds of Formula II and Formula III. These intermediates are useful in the preparation of compounds of Formula I and are as follows: 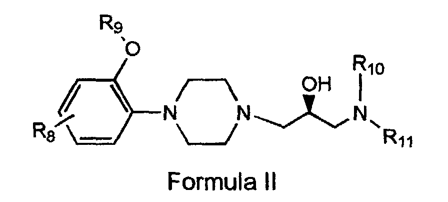 wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₁₀: is hydrogen, C₁₋₅alkoxycarbonyl, phenylC₁₋₅alkoxycarbonyl or allyloxycarbonyl;
- R₁₁: is hydrogen, phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro; 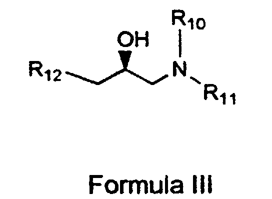
wherein
- R₁₀: is C₁₋₅alkoxycarbonyl, phenylC₁₋₅alkoxycarbonyl, or allyloxycarbonyl;
- R₁₁: is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;
- R₁₂: is halogen, mesyl, tosyl, or hydroxy.

Still further the invention contemplates methods of making compounds of Formula II. Those methods are as follows: Reacting a compound of Formula III 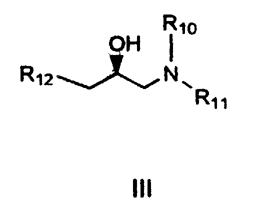 wherein
- R₁₀: is C₁₋₅alkoxycarbonyl, phenylC₁₋₅alkoxycarbonyl, or allyloxycarbonyl;
- R₁₁: is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;
- R₁₂: is halogen, mesyl, tosyl, or hydroxy.
with a piperazine derivative of Formula IV 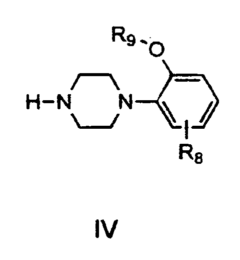 wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
in the presence of a basic reagent to produce a compound for Formula II 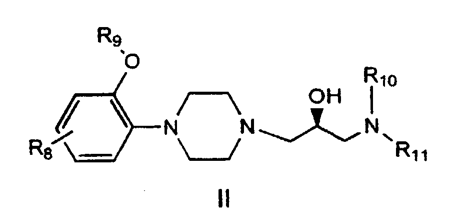 wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₁₀: is C₁₋₅alkoxycarbonyl, phenylC₁₋₅alkoxycarbonyl, or allyloxycarbonyl;
- R₁₁: is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;

### Reacting a compound of Formula II

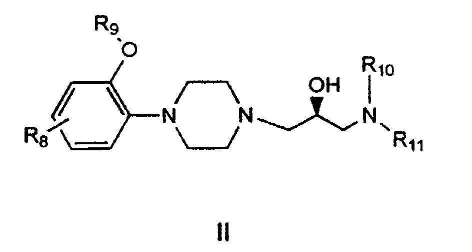

wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₁₀: is C₁₋₅alkoxycarbonyl, phenylC₁₋₅alkoxycarbonyl, or allyloxycarbonyl;
- R₁₁: is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;
with an acidic reagent to give a compound of Formula II 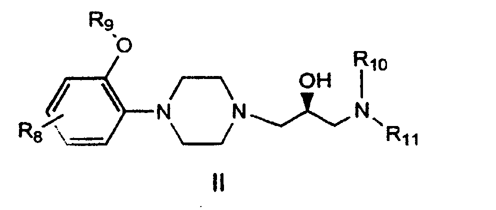 wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₁₀: is hydrogen
- R₁₁: is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;

### Reacting a compound of Formula II

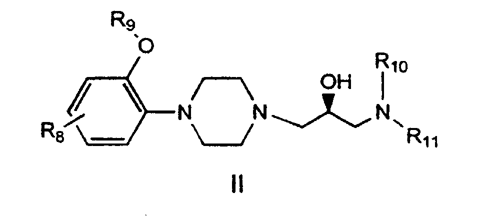

wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₁₀: is hydrogen
- R₁₁: is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;
with a reducing agent to give a compound of Formula II 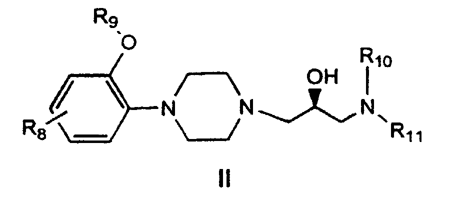 wherein
- R₈: is hydrogen, halogen, C₁₋₅alkoxy, hydroxyl, or C₁₋₅alkyl;
- R₉: is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
- R₁₀: is hydrogen;
- R₁₁: is hydrogen;

### DETAILED DESCRIPTION OF THE INVENTION

The terms used in describing the invention are commonly used and known to those skilled in the art. However, the terms that could have other meanings are defined. "HBSS" refers to Hank's Balanced Salt Solution. "Independently" means that when there are more than one substituent, the substitutents may be different. The term "alkyl" refers to straight, cyclic and branched-chain alkyl groups and "alkoxy" refers O-alkyl where alkyl is as defined supra. "DMAP" refers to dimethylaminopyridine, "TFA" refers to trifluoroacetic acid, "HOBT" refers to hydroxybenzotriazole hydrate, "HATU" refers to O-(7-azabenzotriazol-1-yl)-1,1,3,3-tetrametyluronium hexafluorophosphate, and "EDCI" refers to 1-(3-dimethylaminopropyl)-3-ethylcarbodiimide hydrochloride. The symbol "Ph" refers to phenyl, and "aryl" includes mono and fused aromatic rings such as phenyl and naphthyl. The symbol "CPDA" refers to 1,1-cyclopentanediacetimid-1-yl and "IID" refers to 1H-isoindole 1,3-(2H)dion-1-yl. The symbol "ES" refers to electrospray and the symbol "MS" refers to mass spectrum. Some of the compounds of Formula I include a chiral carbon atom. Therefore those compounds may be prepared as stereoisomers, racemic mixtures or pure enantiomers. All stereoisomers, pure enantiomers and racemic mixtures are considered to be within the scope of this invention.

The compounds of the invention may be prepared by the following schemes, where some schemes produce more than one embodiment of the invention. In those cases, the choice of scheme is a matter of discretion which is within the capabilities of synthetic chemists.

A compound of Formula I where A, B, and E are carbon, R₁ is hydrogen, R₂ is phenyl, R₃ is hydroxy, R₄ is hydrogen, and R₅ is 3-trifluoromethyl may be prepared using Scheme 1. The scheme assembles two halves of the desired molecule and couples them together using peptide coupling reagents. One half is prepared by treating 1,2,4-benzenetricarboxylic anhydride, 1a, with a substituted aniline derivative, 1b, at about 130 °C in an acidic solvent such as glacial acetic acid for about 16-24 h to give the carboxy substituted phthalimido derivative 1c. The other half is prepared in two steps. First, 1-azido-3-(p-toluenesulfonyloxy)propan-2-ol 1d, is heated at about 100 °C with an appropriately substituted piperazine derivative, 1e for about 2-5 days to give the azide 1f. This azide is treated with Pd/C and H₂ (50 psi) in an inert solvent over 16 h to give the free amine 1g. This amine is treated with 1c, HOBT, DMAP, EDCI, and N,N'-diisopropylethylamine in methylene chloride at about room temperature for 2-6 h to give the desired compound of Formula I. Alternatively, 1c and 1g may be coupled using other peptide coupling agents such as HATU and DMAP. This scheme may be used to prepare a number of compounds of Formula I. For example, if compounds where A, B or E is nitrogen are desired, replace 1b with an amino pyridine derivative such as 2-aminopyridine and follow the remaining steps of the scheme. To prepare compounds where R₁ and R₂ vary, simply replace the illustrated 1e with any known substituted piperazines. Although the illustrated product was prepared from the racemic azide 1d, the pure enantiomers of this azide are known and can be used in this scheme.

Compounds where R₃ is carbonyl may be prepared by treating the products of Scheme 1 with an oxidizing agent such as the Swem's reagent (formed with oxalyl chloride and DMSO) at -78 °C to room temperature over 30 min to 1h.

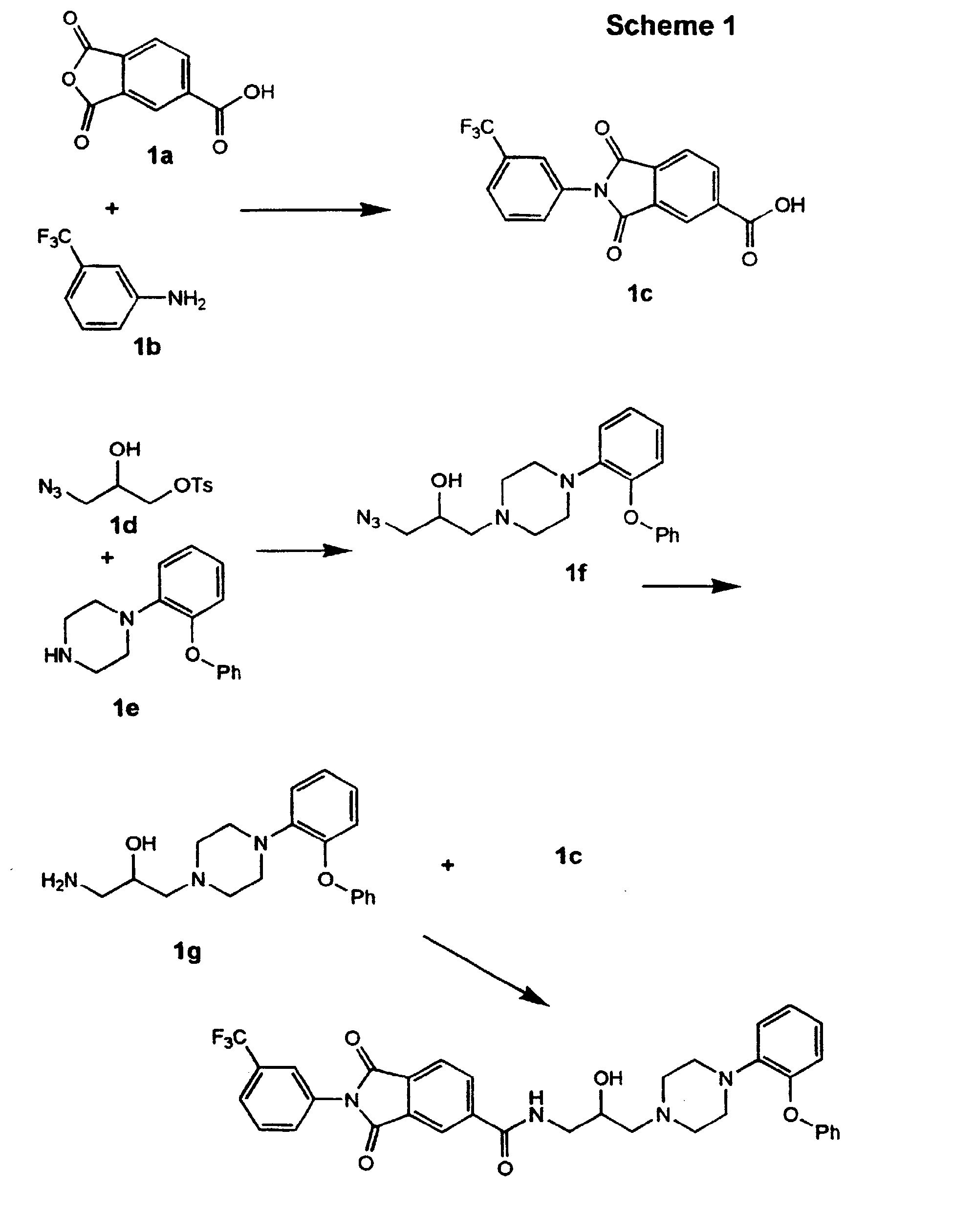

Scheme 2 may be used to prepare compounds of Formula I where A is nitrogen, R₁ is fluoro, R₂ is ethyl, R₃ is hydrogen, R₄ is hydrogen, and R₅ is hydrogen. Treatment of 1,2,4-benzenetricarboxylic anhydride, 2a, with the aniline derivative, 2b gives the phthalimide 2c. An appropriately substituted piperazine derivative 2d is treated with the N-BOC protected 3-bromopropylamine and cesium carbonate in acetonitrile at reflux for 16 h to give the substituted piperazine derivative 2f. This derivative is converted to the free amine, 2g, by treatment with TFA and methylene chloride at room temperature over 2-6 h. Derivatives 2g and 2c were coupled using HOBT, DMAP, EDCI, and N,N'-diisopropylethylamine in methylene chloride at about room temperature for 2-6 h to give the desired compound of Formula I. As described in Scheme 1, Scheme 2 may be modified to give all of the compounds of Formula I.

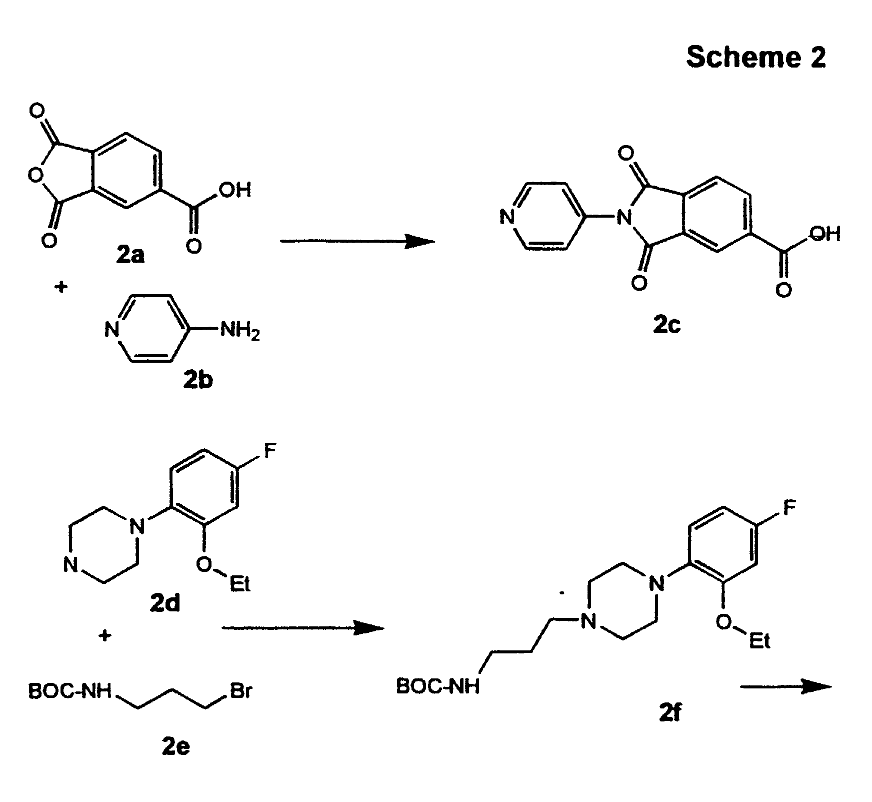

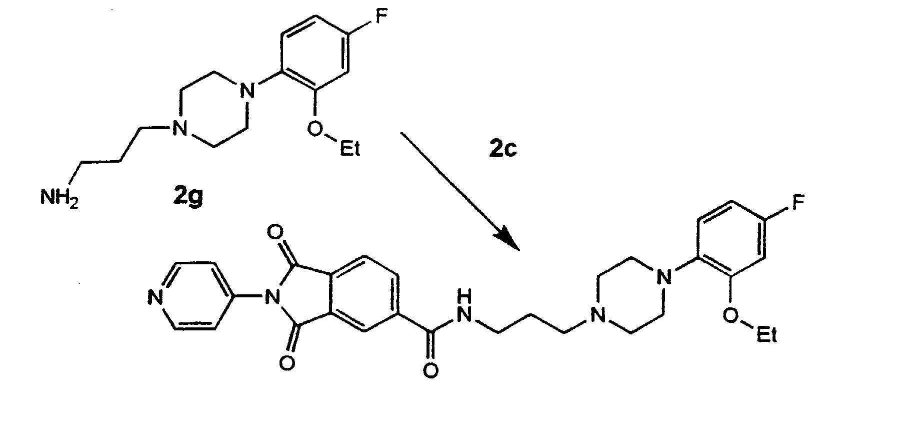

To produce compounds of the invention where R₄ is other than hydrogen, Scheme 3 may be used. The amino group of intermediate 2g may be treated with an aldehyde 3a such as benzaldehyde to give the imine 3b. This intermediate may be reduce with NaBH₄ at room temperature to give the monoamine 3c. This amine is coupled with a substituted phthalimide derivative, 2c using HATU, DMAP and diisopropylethylamine in methylene chloride at about room temperature for 2-6 h to give the desired compound of Formula I. As described in previous schemes, Scheme 3 may be modified to give a number of compounds of Formula I. For example, to produce a compound where R₃ is hydroxy, replace 2g with intermediate 1g and follow the remaining steps of Scheme 3.

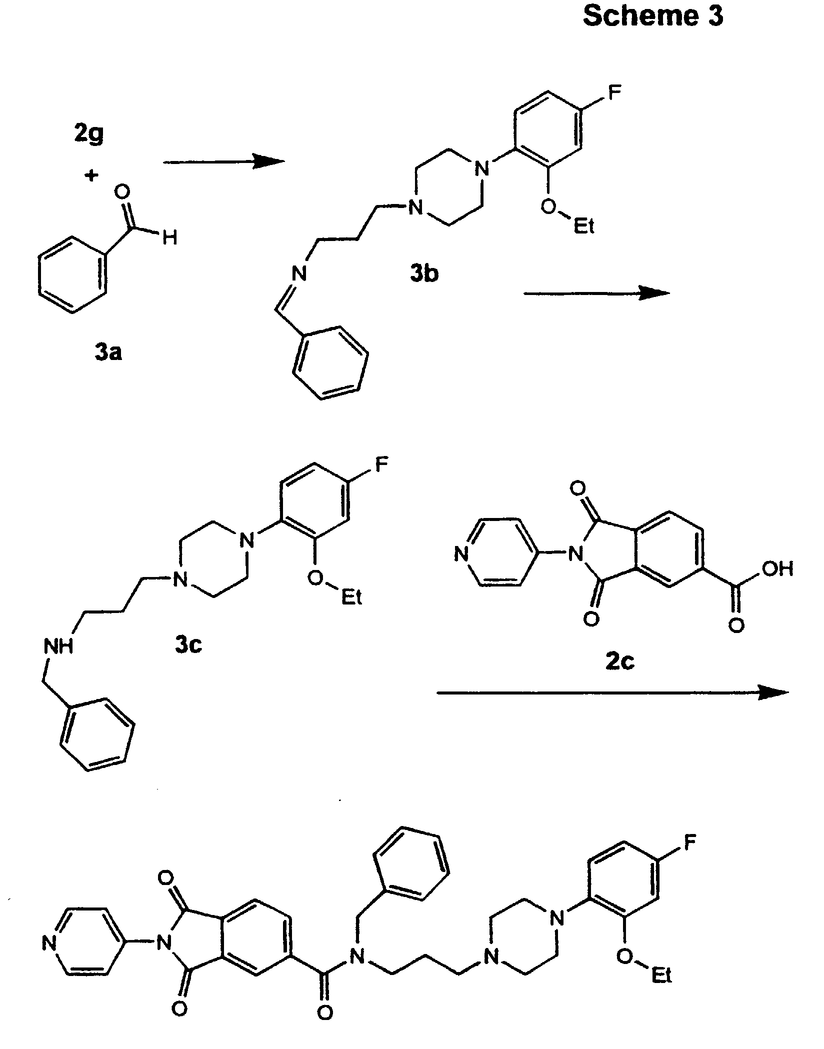

To produce compounds of the invention where R₃ is C₁₋₅alkoxy, Scheme 4 may be used. Treatment of the azido derivative 1d with an appropriately substituted piperazine, such as 4a at about 100 °C for about 2-5 days gives intermediate 4b. This intermediate is treated with two equivalents of a strong organic base, such as sodium hydride in an inert solvent, such as THF, at 0 °C for about 1-5 h; followed by treatment with an additional equivalent of base and an alkylating agent such as ethyl iodide, at 0 °C for about 1-5 h to give the ether 4c. This ether is treated with Pd/C and H₂ (ca. 50 psi) in an inert solvent over 16 h to give the free amine 4d. This amine is coupled with a phthalimido derivative such as 4e with HATU and DMAP to give the desired compounds of the invention. As discussed in schemes 1-3, scheme 4 may be modified in a like manner to give all of the compounds of Formula I.

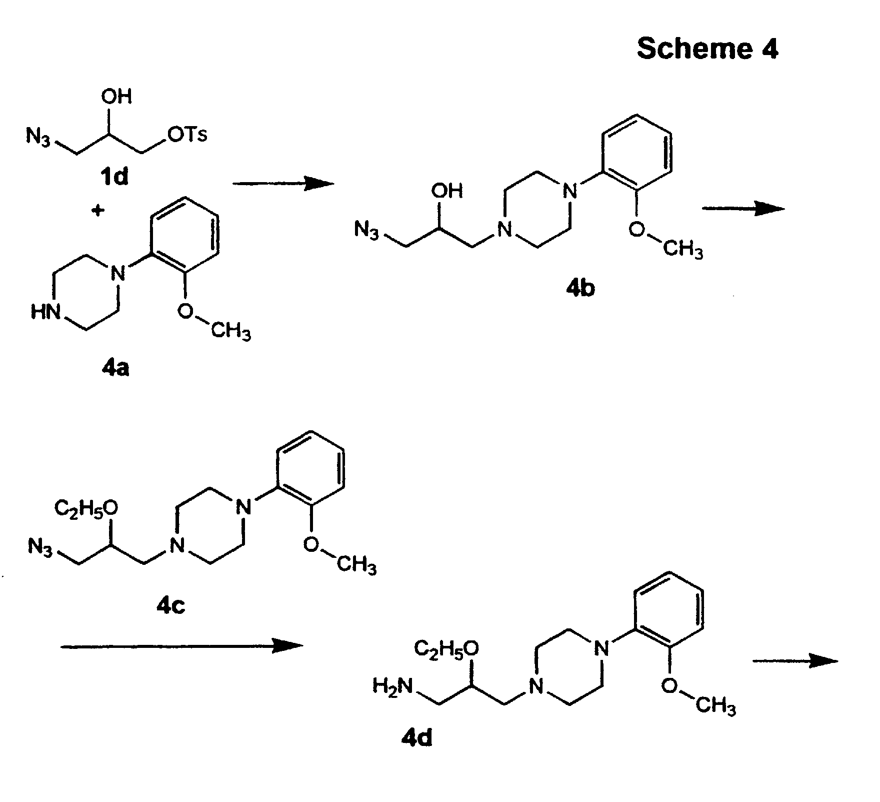

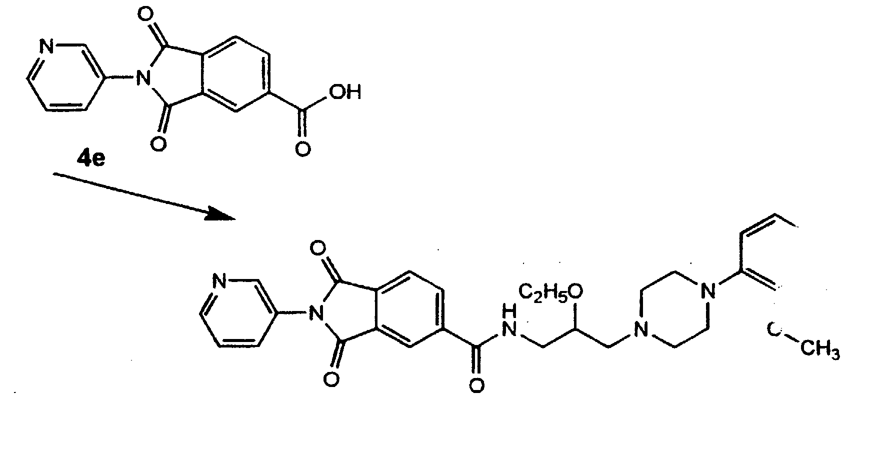

To produce pure enantiomers of compounds of Formula I where R₃ is hydroxy, Scheme 5 may be used. (S)(+) epichlorohydrin (97% ee) may be treated with benzylamine in a suitable organic solvent such as hexane at about room temperature for about 48-72 hours to give hydroxy compound 5a. This intermediate may be treated with a BOC reagent agent such as di-tert-butyl dicarbonate, and an organic base such as triethylamine in an inert solvent such as THF at about 0 °C to about room temperature over 10 to 24 h to give the N-protected derivative 5b. This intermediate may be treated with piperazine derivative, 5c, a basic reagents, such as potassium hydroxide, sodium hydroxide, or lithium hydroxide, in an alcoholic solvent such as methanol at about 0 °C to about room temperature over about 1 to about 3 days to give the coupled derivative 5d. This compound may be deprotected by treatment with an acidic reagents, such as TFA or 1-6N HCI, at about room temperature over 18-24 h to give free amine 5e. This amine may be debenzylated with using a reducing agents, such as palladium catalyst and ammonium formate, sodium in liquid ammonia, or palladium and hydrogen, in an alcoholic solvent such as EtOH at about 45-60 °C over 20 h to give the primary amine 5f. This amine may be coupled to acids of type 5g using peptide coupling agents such as HATU to give a compound of Formula I. As described in Scheme I, Scheme 5 may be modified to give a number of compounds of Formula I.

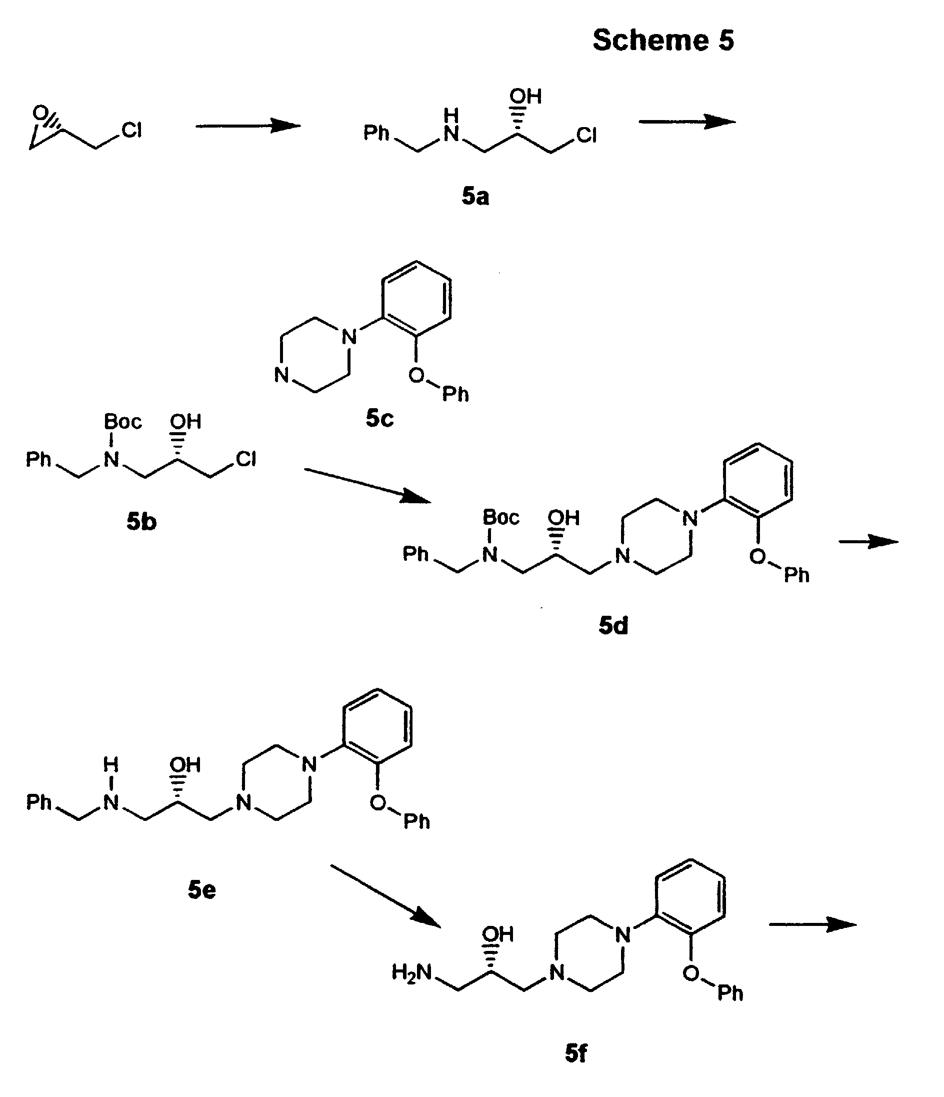

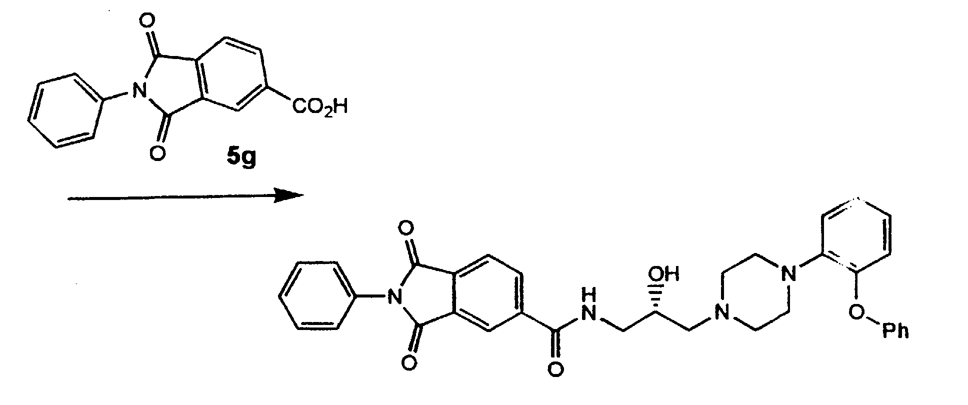

Although the claimed compounds are useful as antagonists of α1ₐ-AR, some compounds are more active than others and are either preferred or particularly preferred. The preferred compounds of Formula I include:
- R₁: is halogen or hydroxy,
- R₂: is phenylC₁₋₅alkyl or hydrogen,
- R₃: is C₁₋₅alkoxy,
- R₄: is C₁₋₅alkyl,
- R₅, R₆, and R₇: are independently selected from hydrogen, nitrile and amino,
- A is: nitrogen or carbon,
- B is: carbon, and
- C: is carbon.
The particularly preferred compounds of Formula I include compounds where:
- R₁: is hydrogen,
- R₂: is C₁₋₆alkyl, phenyl or substituted phenyl,
- R₃: is hydrogen, hydroxy,
- R₄: is hydrogen,
- R₅, R₆, and R₇: are independently selected from halogen, hydrogen, hydroxy, C₁₋₈alkyl, C₁₋₅alkoxy, and diC₁₋₅alkylamino,

- A: is carbon,
- B: is carbon, and
- E: is carbon.
The preferred compounds of Formula II include compound where
- R₈: is hydrogen,
- R₉: is C₁₋₆alkyl,
- R₁₀: is hydrogen, C₁₋₅alkoxycarbonyl, phenylC₁₋₅alkoxycarbonyl, and
- R₁₁: is hydrogen, phenylC₁₋₅alkyl, or C₁₋₅alkoxy substituted phenyl.
The particularly preferred compounds of Formula II include compounds where
- R₈: is hydrogen,
- R₉: is isopropyl,
- R₁₀: is hydrogen, t-butoxycarbonyl, benzyloxycarbonyl, and
- R₁₁: is hydrogen, benzyl.
The preferred compounds of Formula III include compound where
- R₁₀: is C₁₋₅alkoxycarbonyl,
- R₁₁: is phenylC₁₋₅alkyl, or C₁₋₅alkoxy substituted phenyl, and
- R₁₂: is hydrogen or halogen.
The particularly preferred compounds of Formula III include compounds where
- R₁₀: is t-butoxycarbonyl
- R₁₁: is benzyl, and
- R₁₂: is chlorine.
The preferred basic reagent for producing a compound of Formula II is potassium hydroxide. The preferred acidic reagent for treating a compound of Formula III is trifluoroacetic acid. The preferred reducing agent for treating a compound of Formula II is ammonium formate and Pd/C.

As indicated by the biological activity, the compounds of Formula I may be used in pharmaceutical compositions to treat patients (humans and other primates) with disorders related to inhibiting the activity of the α1ₐ adrenergic receptor. The preferred route is oral administration, however compounds may be administered by intravenous infusion. Oral doses range from about 0.01 to about 100 mg/kg daily; where the optimal dose range is about 0.1 to about 25 mg/kg/per day. Infusion doses can range from about 0.001-1 mg/kg/min of inhibitor, admixed with a pharmaceutical carrier over a period ranging from several minutes to several days.

The pharmaceutical compositions can be prepared using conventional pharmaceutical excipients and compounding techniques. Oral dosage forms may be elixers, syrups, capsules tablets and the like. Where the typical solid carrier is an inert substance such as lactose, starch, glucose, methyl cellulose, magnesium sterate, dicalcium phosphate, mannitol and the like; and typical liquid oral excipients include ethanol, glycerol, water and the like. All excipients may be mixed as needed with disintegrants, diluents, granulating agents, lubricants, binders and the like using conventional techniques known to those skilled in the art of preparing dosage forms. Parenteral dosage forms may be prepared using water or another sterile carrier.

Typically the compounds of Formula I are isolated and used as free bases, however the compounds may be isolated and used as their pharmaceutically acceptable salts. Examples of such salts include hydrobromic, hydroiodic, hydrochloric, perchloric, sulfuric, maleic, fumaric, malic, tartatic, citric, benzoic, mandelic, methanesulfonic, hydroethanesulfonic, benzenesulfonic, oxalic, pamoic, 2-naphthalenesulfonic, p-toluenesulfonic, cyclohexanesulfamic and saccharic.

In order to illustrate the invention the following examples are included. These examples do not limit the invention. They are meant only to suggest a method of practicing the invention. Those skilled in the art may find other methods of practicing the invention, which are obvious to them. However those methods are deemed to be within the scope of this invention.

### PREPARATIVE EXAMPLES

### Example 1

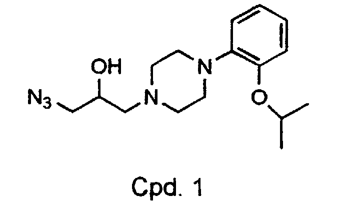

The fumarate salt of 1-(2-isopropoxyphenyl)-piperazine (3.91 g, 12 mmol) was basified with 20% NaOH _{(aq)} (100 mL) and extracted with methylene chloride. The combined organic layers were dried (Na₂SO₄) and concentrated to give an oil (2.74 g). A mixture of the oil and 1-azido-3-(p-toluenesulfonyloxy)propan-2-ol (3.25 g, 12 mmol, Antonin Holy, Collect. *Czech. Chem. Comm*. **1989,** *54*(2), 446) was stirred at 100 °C for 36 h. The cooled mixture was diluted with water and extracted with ether, dried (Na₂SO₄) and concentrated. The product was purified by column chromatography (SiO₂) to yield compound 1 as (2.92 g, 76%) as a light-brown solid: MS (ES) m/z: 320 (MH⁺); Anal. Calcd for C16H25N5O2:C, 60.17; H, 7.89; N, 21.93. Found: C, 60.45; H, 7.83; N, 22.01.

### Example 2

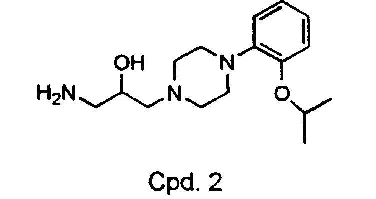

10% HCI (6 mL) was added to a mixture of compound 1 (2.43 g, 7.6 mmol) and 10% Pd/C (1.22 g) in MeOH (60 mL) and the mixture was hydrogenated under H₂ (50 psi) in a Parr shaker for 16 h at 20°C. The mixture was filtered through celite and the filtrate was concentrated. The residue was basified with 20% NaOH and extracted with methylene chloride. The combined organic layers were dried (Na₂SO₄) and concentrated to yield compound 2 as a yellowish oil (2.2 g, 95%): MS (ES) m/z: 294 (MH⁺)

### Example 3

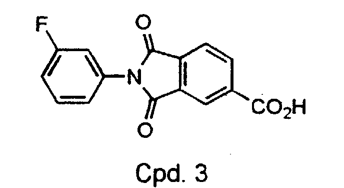

A mixture of the 1,2,4-benzenetricarboxylic anhydride (10 g, 52 mmol) and 3-fluoroaniline (5.77 g, 52 mmol) in glacial acidic acid (200 mL) was stirred at 130 °C for 16 h. The light-brown solution was cooled to 20 °C to give a yellow solid precipitate. The yellow solid was collected via filtration and was washed thoroughly with water to remove the trace amount of acetic acid. The product was dried at 60 °C for 36 h under vacuum to yield a yellow solid (11.41 g, 77%): MS (ES) m/z: 284 (MH⁺).

### Example 4

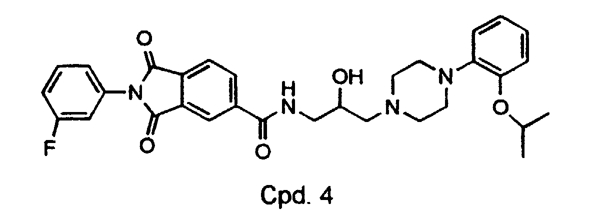

A mixture of compound 2 (226 mg, 0.77 mmol), compound 3 (220 mg, 0.77 mmol), EDCI (151 mg, 0.78 mmol), HOBT (105 mg, 0.78 mmol), DMAP (cat.) and N,N-diisopropylethylamine (0.52 mL) in methylene chloride (6 mL) was stirred at 20 °C for 3 h. The mixture was concentrated, diluted with water and extracted with EtOAc. The combined organic layer was dried (Na₂SO₄), concentrated. The product was purified by column chromatography (SiO₂) and further recrystallized from EtOAc/Hexane to yield compound 4 (101 mg, 23%) as a yellow solid: ¹H NMR (300 MHz, CDCl₃) δ 8.34 (s, 1 H), 8.30 (d, J = 7.8 Hz, 1 H), 8.04 (d, J = 7.8 Hz, 1 H), 7.48 (m, 1 H), 7.26 (m, 1 H), 7.14 (m, 1 H), 6.91 (m, 6 H), 4.59 (m, 1 H), 4.02 (m, 1 H), 3.79 (m, 1 H), 3.45 (m, 1 H), 3.13 (m, 4 H), 2.87 (m, 2 H), 2.62 (m, 2 H), 2.50 (m, 2 H), 1.34 (d, J = 6.0 Hz, 6 H); MS (ES) m/z: 561(MH⁺).

### Example 5

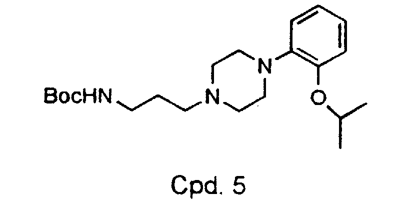

3-Bromopropylamine hydrobromide (5 g, 22.8 mmol) was dissolved in 10% NaOH (50 mL), extracted with methylene chloride and concentrated . To the free base in methylene chloride was added (Boc)₂O (5.23 g, 23.9 mmol) and this mixture was stirred at 20 °C for 4h. The methylene chloride layer was washed with H₂O, diluted citric acid (6%), NaHCO₃ and sat NaCI solution, dried and concentrated. The product was purified by column chromatography (SiO₂) to yield the protected amine (4.84g, 89%). The fumarate salt of 1-(2-isopropoxyphenyl)-piperazine (5.1g, 15 mmol) was basified with 20% NaOH _{(aq)} (100 mL),extracted with methylene chloride, dried (Na₂SO₄) and concentrated to give a yellow oil (3.15 g). A mixture of the oil,the protected amine (3.42 g, 14.3 mmol), and Cs₂CO₃ (4.66 g, 14.3 mmol) in CH₃CN (50 mL) was heated at reflux overnight. Solid was filtered off and the filtrate was evaporated. The product was purified by column chromatography (SiO₂) to yield compound 5(4.4 g, 81%): MS (ES) m/z: 378(MH⁺) 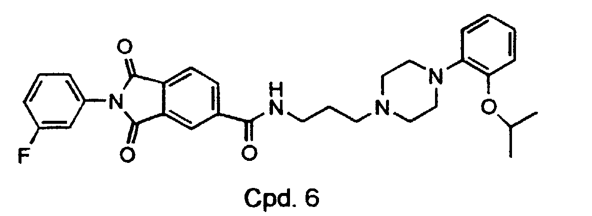 Compound 5 (3.97 g, 11.4 mmol) was dissolved in 25% TFA/methylene chloride (50 mL). The reaction was stirred at RT for 5 h, the solvent was evaporated and solid residue was obtained. This solid was dissolved in 20% NaOH (aq) (100 mL) and stirred for 40 min. Then the free base was extracted into methylene chloride (3x). A light yellow oil was obtained (3.0 g, 95%). A solution of this free amine (3.0 g, 10.8 mmol) and diisopropylethyl amine (5.6 g, 43.3 mmol) in methylene chloride (80 mL) was added to a mixture containing EDCI (2.08 g, 10.8 mmol) HOBT (1.46 g, 10.8 mmol), catalytic amount of DMAP and compound 3 (3.09 g, 10.8 mmol). Reaction was stirred overnight at 20 °C under N₂. The reaction mixture was washed with water (3x). The product was purified by column chromatography (SiO₂) to yield compound 6 (1.34g, 23%): ¹H NMR (300 MHz, CDCl₃) δ 8.34 (m, 2 H), 7.99 (d, J = 8.1 Hz, 1 H), 7.46 (q, J = 8.0, 6.4 Hz, 1 H), 7.18 (m, 2 H), 6.89 (m, 4 H), 4.57 (q, J = 12.0, 6.0 Hz, 1H), 3.67(m, 2 H), 3.09 (brs, 4 H), 2.71 (m, 6 H), 1.87 (m, 2 H), 1.33 (d, J = 6.1 Hz, 6 H); MS (ES) m/z: 545 (MH⁺). Anal. Calc'd for C₃₁H₃₃FN₄O₄: C, 68.37; H, 6.11; N, 10.29. Found: C, 68.13; H, 6.10; N, 10.17

### Example 7

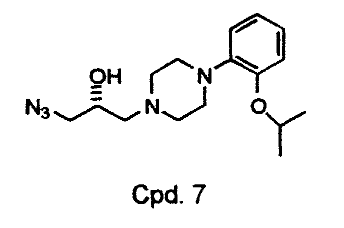

The fumarate salt of 1-(2-isopropoxyphenyl)-piperazine (112.5 g, 345 mmol) was basified with 20% NaOH _{(aq)} (500 mL) and extracted with methylene chloride (3x). The combined organic extracts were dried (Na₂SO₄) and concentrated to give about 70 g oil. A mixture of the oil and (2S)-3-azido-2-hydroxypropyl p-toluenesulfonate (91 g, 335 mmol, Kristina Juricova, *Collect. Czech. Chem. Comm.* **1995,** *60,* 237) was stirred at 100 °C in NMP in the presence of triethylamine (70 g, 690 mmol) for 30 h. The cooled mixture was diluted with water and extracted with ether (3 x 500 mL), back washed once with NaCI (sat) (100 mL), dried (Na₂SO₄) and concentrated. The product was purified by column chromatography (SiO₂) to yield compound 7 (70.6g, 66%) (98.8% ee assay by chiralcel AD column) as a off-white solid after recrystallization from methylene chloride/ hexane: [α]_{D}²⁵ -3.6 ° (c=1, CH₃OH); ¹H NMR (300 MHz, CDCl₃) δ 6.91 (m, 4 H), 4.59 (m, 1 H), 3.93 (m, 1 H), 3.67 (brs,1 H), 3.42 (dd, J = 12.6, 3.8 Hz, 1 H), 3.23 (dd, J = 12.6, 5.4 Hz, 1 H), 3.12 (m, 4 H), 2.83 (m, 2 H), 2.53 (m, 3 H), 2.42 (dd, J = 12.2, 3.8 Hz, 1 H), 1.34 (d, J = 6.0 Hz, 6 H); MS (ES)m/z: 320 (MH⁺)

### Example 8

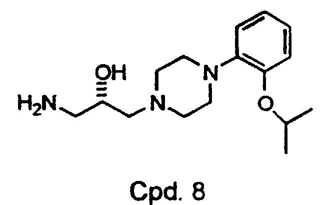

10% HCI (6 mL) was added to a mixture of compound 7 (15 g, 47 mmol) and 10% Pd/C (4 g) in MeOH (100 mL) and the mixture was hydrogenated under H₂ (50 psi) in a Parr shaker for 21 h at 20°C. The resulting mixture was filtered through celite and the filtrate was concentrated. The residue was basified with 20% NaOH_{(aq)} (75 mL) and extracted with methylene chloride (3x), dried (Na₂SO₄) and concentrated to yield compound 8 as a yellowish oil (14 g ,∼100%): [α]_{D}²⁵ +23.6 ° (c=1, CHCl₃); ¹H NMR (300 MHz, CDCl₃) δ 6.91 (m, 4 H), 4.59 (m, 1 H), 3.76 (m, 1 H), 3.12 (m, 4 H), 2.83 (dd, J = 12.7, 3.7 Hz, 2 H), 2.82 (m, 1 H), 2.25-2.68 (m, 8 H), 1.34 (d, J = 6.1 Hz, 6 H); MS (ES) m/z: 294 (MH⁺)

### Example 9

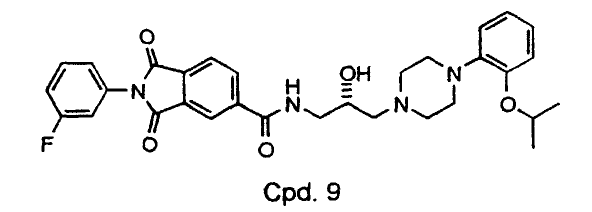

Compound 8 (1 g, 3.41 mmol) was dissolved in a mixture of diisopropylethyl amine (2.3 mL , 13.6 mmol) and methylene chloride (10 mL). To the above light-yellowish solution was added compound 3 (970 mg, 3.4 mmol) and HATU (1.296 g, 3.41 mmol) and stirred at 20°C for 18 h, concentrated. 10% K₂CO₃ (aq) was added and the mixture was extracted with ether (3 x), dried (Na₂SO₄), and concentrated. The product was purified by column chromatography (SiO₂, EtOAc/Hexane then methylene chloride / Acetone) to give an a oily solid. The product was further recrystallized from EtOAc /hexane to give compound 9 as a yellow solid (830 mg, 43%): [α]_{D}²⁵ +8.3 ° (c=1, CHCl₃); ¹H NMR (300 MHz, CDCl₃) δ 8.34 (s, 1 H), 8.30 (d, J = 7.8 Hz, 1 H), 8.04 (d, J = 7.8 Hz, 1 H), 7.48 (m, 1 H), 7.26 (m, 1 H), 7.14 (m, 1 H), 6.91 (m, 6 H), 4.59 (m, 1 H), 4.02 (m, 1 H), 3.79 (m, 1 H), 3.45 (m, 1 H), 3.13 (m, 4 H), 2.87 (m, 2 H), 2.62 (m, 2 H), 2.50 (m, 2 H), 1.34 (d, J = 6.0 Hz, 6 H); MS (ES) m/z: 561(MH⁺).

### Example 10

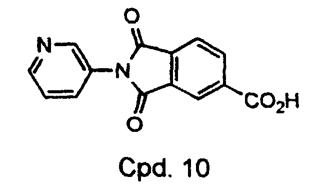

A mixture of the 1,2,4-benzenetricarboxylic anhydride (2 g, 10.4 mmol), and 3-aminopyridine (0.98 g, 10.4 mmol) in glacial acidic acid (40 mL) was stirred at 130 °C for 16 h. The mixture was cooled and the white solid was filtered off and was washed with water. The product was dried for 24 h under vacuum to yield compound 10 as a white solid (2.55 g, 91%): MS (ES) m/z: 267 (MH⁺).

### Example 11

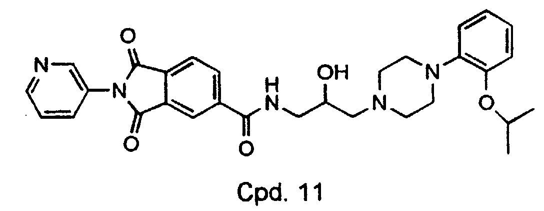

A mixture of compound 2 ( 0.2 g, 0.68 mmol), EDCI (132 mg, 0.68 mmol), HOBT (94 mg, 0.68 mmol), DMAP (cat.), compound 10 (0.18 g, 0.68 mmol) and N,N-diisopropylethylamine (0.46 mL, 2.72 mmol) in methylene chloride (6 mL) was stirred at 20 °C overnight. The mixture was concentrated and purified by column chromatography (SiO₂, methylene chloride/acetone = 10:1 to 1:1) to yield compound 11 as a solid (67 mg, 18%):MS (ES) m/z: 544(MH⁺).

### Example 12

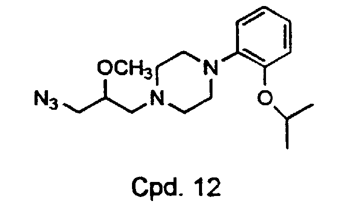

Compound 1 (0.8 g, 2.5 mmol) was dissolved in anhydrous THF (50 mL). The solution was cooled to 0 °C and 60% NaH (0.2 g, 5.0 mmol) was added. The solution was stirred for 10 min and CH₃I (0.53 g, 3.8 mmol) was added. The reaction mixture was stirred at 0 °C for 2 h, NaH (0.1 g, 2.5 mmol) and CH₃I (0.15 mL) were added and this mixture was stirred for another 2 h. The reaction was quenched with sat NH₄Cl, the solvent was evaporated and the aqueous layer was washed with methylene chloride (3x), dried (Na₂SO₄) and concentrated. The product was purified by column chromatography (silica gel) to yield compound 12 (0.69 g, 83%): MS (ES) m/z: 334 (MH⁺).

### Example 13

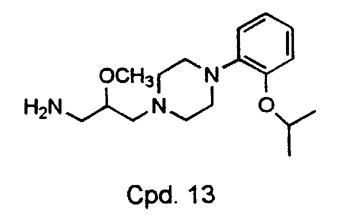

10%HCI (0.3 mL) was added to a mixture of compound 12 (0.64 g, 1.9 mmol) and 10% Pd/C (0.13 g) in MeOH (5 mL). This mixture was hydrogenated under H₂ (50 psi) in a Parr shaker overnight, filtered through celite and the filtrate was concentrated. The residue was basified with 20% NaOH and extracted with methylene chloride (3x), dried (Na₂SO₄) and concentrated to give a yellow oil at quantitative yield. MS (ES) m/z: 308 (MH⁺).

### Example 14

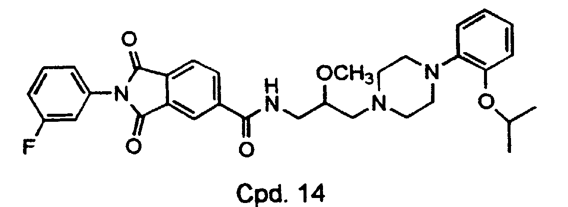

Compound 13 (0.15 g, 0.49 mmol) was dissolved in methylene chloride (4 mL) and 4 eq of diisopropylethyl amine (0.25 g, 1.95 mmol) was added. To this solution was added a mixture of HATU, (0.185 g, 0.49 mmol) and compound 3 (0.14 g, 0.49 mmol) and the reaction was stirred overnight under N₂ at RT. The solvent was evaporated and the product was purified by flash chromatography (SiO₂, methylene chloride/Acetone = 10:1, 8:1, 6:1, 4:1, 2:1). The product was recrystallized from EtOAc/hexane to yield light yellow solid 0.08 g (29%): ¹H NMR (300 MHz, CDCl₃) δ 8.36 (m, 2 H), 8.18 (brs, 1 H), 8.02 (d, J =7.69 Hz, 1 H), 7.47 (m, 1 H), 7.22 (m, 3 H), 7.00 (m, 1 H), 6.87 (m, 3 H), 4.57 (m, 1 H), 3.76 (m, 3 H), 3.50 (s, 3 H), 3.22 (m, 10 H), 1.35 (d, J =6.0 Hz, 6 H); MS (ES) m/z: 575 (MH⁺).

### Example 15

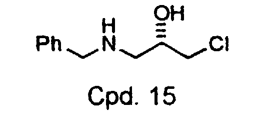

A mixture of (S)-(+)-epichlorohydrin (10 g, 108.1 mmol, Aldrich, 97% ee) and benzylamine (11.57 g, 108.1 mmol) in hexane (40 mL) were stirred at 20 °C for 62 h. White solid precipitated. More hexane (∼ 350 mL) added, stirred for 20 min. and sonicated to break the big chunks of white solid. The white solid was collected by filtration and washed with hexane, dried under vacuum to give 19.8 g (92%) white solid. The white solid was recrystallized from EtOAc/hexane to give 17.76 g (82%) of 15 as a white crystalline solid; 1H NMR (300 MHz, CDCl₃) δ 7.31 (m, 5 H), 3.88 (m, 1 H), 3.79 (m, 2 H), 3.53 (d, J = 5.3 Hz, 2 H), 2.89 (m, 2 H), 2.81 (dd, J = 12.4, 4.1 Hz, 1 H), 2.69 (dd, J = 12.2, 7.9 Hz, 1 H); MS (ES): 200 (MH⁺); Anal. Calcd. for C₁₀H₁₄NOCl: C, 60.15; H, 7.07; N, 7.01. Found: C, 60.10; H, 7.02; N, 6.92.

### Example 16

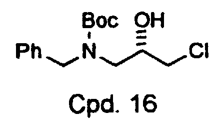

Boc₂O (11 g, 50.1 mmol) and triethylamine (10.12 g, 100 mmol) were dissolved in THF (25 mL) and cooled to 0 °C . The amine 15 (10 g, 50.1 mmol) was added in portions and stirred for 20 h while the temperature was warmed up to 20 °C overnight. The solvent was concentrated in vacuo and water wasadded. The mixture was extracted with ether (3x), dried (Na₂SO₄) and concentrated. The crude residue was recrystallized from EtOAc/hexane to give 9.9 g (66%) of 16 as a white crystalline solid. The filtrate was concentrated (3.1 g as oil) and more product was purified by column chromatography (short column, 8 cm height of SiO₂, EtOAc/hexane as solvent). The oil was recrystallized from EtOAc/hexane to give another 2.78 g (18%) of 16 as a white crystaline solid; [α]_{D}²⁵ = -10.2° (c = 1, CHCl₃); ¹H NMR (300 MHz, CDCl₃) δ 7.22-7.36 (m, 5 H), 4.52 (m, 2 H), 4.30 (brs, 0.5 H), 3.96 (m, 1 H), 3.36-3.97 (m, 4 H), 1.47 (s, 9 H); MS (ES): 322 (M+Na); Anal. Calcd. for C₁₅H₂₂NO₃Cl: C, 60.10; H, 7.40; N, 4.67. Found: C, 60.26, H, 7.42; N, 4.63

### Example 17

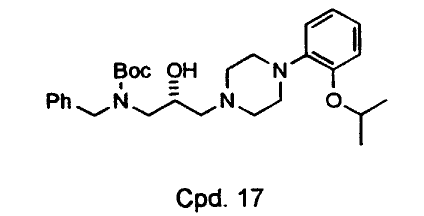

KOH (11.23 g, 200.5 mmol) was dissolved in methanol (280 mL), and the fumarate salt of 1-(2-isopropoxyphenyl)-piperazine (10.9 g, 33.4 mmol) was added and stirred at 20 °C for 20 min then cooled to 0 °C . The Boc-protected amine 16 (10 g, 33.4 mmol) was added to the methanol solution at 0 °C and stirred for 20 h while the temperature was warm up to 20 °C overnight. The solvent was removed, water was added and the mixture was extracted with ether (3x), dried (Na₂SO₄) and concentrated. The product was purified by column chromatography (short column, 8 cm height SiO₂, EtOAc/hexane as solvent) to give 10.22 g (63%) of 17 (-100% ee, Chiralpak OD 4.6 x 250 mm. 1 mUmin, 254 nm, mobile phase: 90/10/0.1 of hexane/IPA/ 0.1% diethylamine) as a yellowish oil; ¹H NMR (300 MHz, CDCl₃) δ 7.26-7.35 (m, 5 H), 6.91 (m, 4 H), 4.68 (d, J = 15.6 Hz, 1 H), 4.59 (m, 3 H), 3.95 (m, 1 H), 3.35 (m, 2 H), 3.11 (m, 4 H), 2.75 (m, 2 H), 2.54 (m, 2 H), 2.38 (m, 2 H), 1.45 (m, 9 H), 1.34 (d, J = 6.1 Hz, 6 H); MS (ES): 484 (MH⁺).

### Example 18

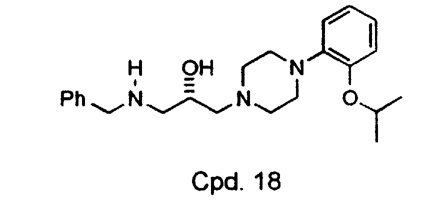

A mixture of compound 17 (233 mg, 0.48 mmol) and 25% TFA/methylene chloride (3 mL) was stirred at 20 °C for 18 h. The solvent was concentrated in vacuo and the residue was basified with 20% NaOH (aq), extracted with methylene chloride (3x), dried (Na₂SO₄) and concentrated to give 174 mg (∼95%) of 18 as an oil which was used directly without further purification; MS (ES): 384 (MH⁺).

### Example 19

### Alternative Preparation of Cpd. 8

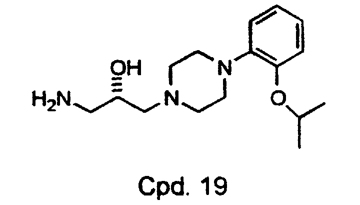

To a mixture of 18 (-154 mg, 0.4 mmol) and 10% Pd/C (154 mg) in EtOH (3 mL) was added ammonium formate (151 mg, 2.4 mmol) and stirred at 55-60 °C for 20 h. The mixture was filtered thru celite and washed with methanol. The filtrate was concentrated. The product was purified by a short column (5 cm height of SiO₂) to give 63 mg (54%) of 19 as a oil; [α]_{D}²⁵ +23.6° (c = 1, CHCl₃); ¹H NMR (300 MHz, CDCl₃) δ 6.91 (m, 4 H), 4.59 (m, 1 H), 3.76 (m, 1 H ),3.12(m, 4 H),2.83(dd, J = 12.7,3.7Hz, 2 H),2.82(m, 1 H), 2.25-2.68 (m, 8 H), 1.34 (d, J = 6.1 Hz, 6 H); MS (ES): 294 (MH⁺).

### Example 20

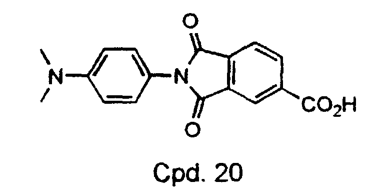

A mixture of the 1,2,4-benzenetricarboxylic anhydride (7 g, 36.4 mmol), and N,N-dimethyl-1,4-phenylenediamine (4.96 g, 36.4 mmol) in glacial acidic acid (120 mL) was stirred at 130°C for 16 h. The reaction solution was cooled to 20 °C and light brown solid precipitated out. The solid was collected thru filtration and was washed thoroughly with water to remove the trace amount of acetic acid. The product was dried at 40 °C for 36 h under vacuum to yield 8.0 g (71%) of 20 as a light brown solid: ¹H NMR (300MHz, DMSO-d₆) δ 8.40 (d, J = 8.5 Hz, 1 H), 8.28 (s, 1 H), 8.05 (d, J = 7.8 Hz, 1 H), 7.22 (d, J = 8.9 Hz, 2 H), 6.82 (d, J = 8.9 Hz, 2 H), 2.96 (s, 6 H); MS (ES): 309 (MH⁺).

### Example 21

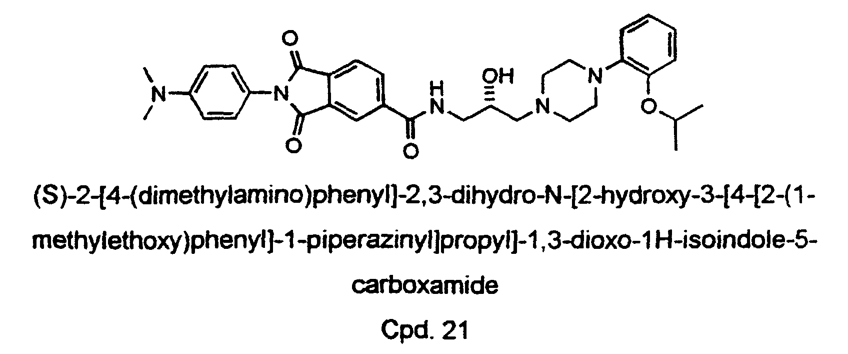

The piperazine 19 (0.4 g, 1.36 mmol) was dissolved in a mixture of diisopropylethyl amine (0.7 g, 5.46 mmol) and methylene chloride (10 mL). To the above solution was added compound 20 (420 mg, 1.36 mmol) and HATU (0.52 g, 1.36 mmol) and stirred at 20°C for 18 h. The reaction mixture was washed with 3% K₂CO₃ (aq) and the organic layer was dried (Na₂SO₄), and concentrated. The product was purified by column chromatography (SiO₂, CH₂Cl₂ / Acetone = 10:1, 8:1, 6:1, 4:1, 2:1) to give 0.33 g (41%) of cpd. 21 as a light brown solid: ¹H NMR (300 MHz, CDCl₃) δ 8.27 (m, 2 H), 8.00 (d, J = 7.7 Hz, 1 H), 7.23 (s, 1 H), 6.90 (m, 5 H), 6.79 (d, J = 8.9 Hz, 2 H), 4.59 (m, 1 H), 4.00 (m, 1 H), 3.83 (m, 1 H), 3.44 (m, 1 H), 3.12 (brs, 4 H), 3.00 (s, 6 H), 2.85 (m, 2 H), 2.52 (m, 4 H) 1.34 (d, J = 6.1 Hz, 6 H); MS (ES): 586 (MH⁺) [α]_{D}²⁵= 9.6 °(C= 0.2, CHCl₃)

### BIOLOGICAL EXAMPLES

The biological activity and selectivity of the compounds of the invention was demonstrated by the following assays. The first assay tested the ability of compounds of Formula I to bind to membrane bound receptors α1ₐ-AR, α1_{b}-AR and α1_{d}-AR.

### Example 22

The DNA sequences of the three cloned human α1-AR subtypes have been published. Furthermore, the cloned cDNAs have been expressed both transiently in COS cells and stably in a variety of mammalian cell lines (HeLa, LM(tk-), CHO, rat -1 fibroblast) and have been shown to retain radioligand binding activity and the ability to couple to phosphoinositide hydrolysis. We used published DNA sequence information to design primers for use in RT-PCR amplification of each subtype to obtain cloned cDNAs. Human poly A+ RNA was obtained from commercially available sources and included hippocampus and prostate samples, sources which have been cited in the literature. For the primary screen a radioligand binding assay was used which employed membrane preparations from cells expressing the individual cloned receptor cDNAs. Radiolabeled ligands with binding activity on all three subtypes (non-selective) are commercially available ([125I]-HEAT, [3H]-prazosin). Each α1 receptor subtype was cloned from poly A+ RNA by the standard method of reverse transcription-polymerase chain reaction (RT-PCR). The following sources of polyA+ RNA were used for the cloning of the α1 receptor subtypes: α1ₐ-AR, human hippocampus and prostate, α1_{b}-AR, human hippocampus, α1_{d}-AR, human hippocampus. The resulting cDNAs were cloned into the pcDNA3 mammalian expression vector (Invitrogen Corp., San Diego CA). Each DNA was sequenced for verification and to detect any possible mutations introduced during the amplification process. Any deviation in sequence from the published consensus for each receptor subtype was corrected by site-directed mutagenesis.

The three α1-AR subtypes (a, b, d) were transfected into COS cells using a standard DEAE-dextran procedure with a chloroquine shock. In this procedure, each tissue culture dish (100mm) was inoculated with 3.5 x 10⁶ cells and transfected with 10 µg of DNA. Approximately 72 hours post-transfection, the cells were harvested and COS membranes were prepared. Transfected COS cells from 25 plates (100mm) were scraped and suspended in 15mL of TE buffer (50mM Tris-HCI, 5mM EDTA, pH7.4). The suspension was disrupted with a homogenizer. It was then centrifuged at 1000xg for 10 minutes at 4 °C. The supernatant was centrifuged at 34,500xg for 20 minutes at 4 °C. The pellet was resuspended in 5mL TNE buffer (50mM Tris-HCI, 5mM EDTA, 150mM NaCI, pH7.4). The resulting membrane preparation was aliquoted and stored at -70 °C. The protein concentration was determined following membrane solubilization with TritonX-100.

The ability of each compound to bind to each of the α1-AR subtypes was assessed in a receptor binding assay. [125I]-HEAT, a non-selective α1-AR ligand, was used as the radiolabeled ligand. Each well of a 96-well plate received: 140 µL TNE, 25 mL [125I]-HEAT diluted in TNE (50,000 cpm; final concentration 50 pM), 10 µL test compound diluted in DMSO (final concentration 1 pM-10 µM), 25 mL COS cell membrane preparation expressing one of the three α1-AR subtypes (0.05-0.2 mg membrane protein). The plate was incubated for 1 hour at room temperature and the reaction mixtures were filtered through a Packard GFIC Unifilter filter plate. The filter plate was dried for 1 hour in a vacuum oven. Scintillation fluid (25 mL) was added to each well, and the filter plate was counted in a Packard Topcount scintillation counter. Data was analyzed using GraphPad Prism software.

Tables A through D list the IC₅₀ values expressed in nanomolar concentration for select compounds of the invention in all receptor subtypes.

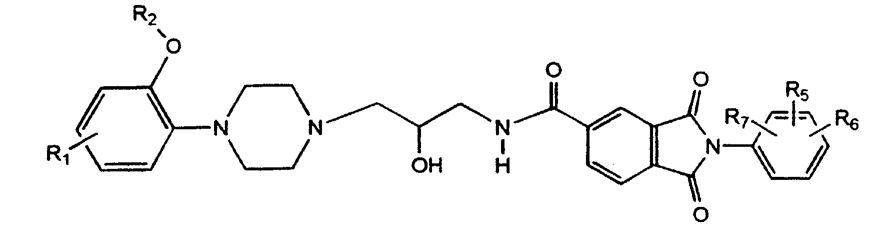

**TABLE A**

| Cpd. | R₁ | R₂ | R₅ | R₆ | R₇ | α1a | α1b | α1d |
|---|---|---|---|---|---|---|---|---|
| 4 | H | i-propyl | 3-F | H | H | 1.5 | 1835 | 76 |
| 48 | H | i-propyl | 4-acetyl | H | H | 1.0 | >2000 | 60 |
| 49 | H | i-propyl | 3-CH₃ | H | H | 0.9 | >2000 | 73 |
| 50 | H | i-propyl | 4-F | H | H | 1.5 | >2000 | 111 |
| 51 | H | i-propyl | H | H | H | 0.9 | >2000 | 65 |
| 52 | H | i-propyl | 4-CH₃ | H | H | 0.66 | >2000 | 62 |
| 53 | H | i-propyl | 4-Cl | H | H | 0.95 | 606 | 55 |
| 54 | H | i-propyl | 3-Cl | H | H | 0.73 | 669 | 37 |
| 55 | H | i-propyl | 4-OCH₃ | H | H | 0.77 | >2000 | 51 |
| 56 | H | i-propyl | 3-Cl | 4-Cl | H | 0.81 | 1225 | 40 |
| 57 | H | i-propyl | 3-CF₃ | H | H | 0.74 | >2000 | 89 |
| 58 | H | i-propyl | 4-OH | H | H | 0.88 | >2000 | 28 |
| 26 | H | i-propyl | 2-OCH₃ | H | H | 1.6 | 1639 | 74 |
| 27 | H | i-propyl** | 3-F | H | H | 8 | >2000 | 63 |
| 9 | H | i-propyl* | 3-F | H | H | 1.0 | >2000 | 190 |
| 28 | H | i-propyl | 4-N(CH₃)₂ | H | H | 0.80 | >2000 | 44 |
| 29 | H | i-propyl | 3-F | 5-F | H | 0.89 | 886 | 38 |
| 30 | H | i-propyl | 4-NO₂ | H | H | 1.8 | >2000 | 38 |
| 31 | H | i-propyl** | 4-OCH₃ | H | H | 2.2 | >2000 | 52 |
| 32 | H | i-propyl* | 4-OCH₃ | H | H | 0.23 | 1750 | 124 |
| 21 | H | i-propyl* | 4-N(CH₃)₂ | H | H | 0.36 | >2000 | 52 |
| 34 | H | i-propyl* | 4-CH₃ | H | H | 0.16 | 1650 | 37 |
| 35 | H | i-propyl* | 4-OH | H | H | 0.46 | >2000 | 137 |
| 36 | H | i-propyl* | 2-OCH₃ | H | 4-OCH₃ | 0.59 | >2000 | 56 |
| 37 | H | i-propyl* | 5-OCH₃ | 3-OCH₃ | 4-OCH₃ | 0.62 | >2000 | 51 |
| 38 | H | i-propyl* | H | 3-OCH₃ | 4-OCH₃ | 0.93 | >2000 | 175 |
| 39 | H | i-propyl | H | 3-CH₃ | -4-CH₃ | 0.44 | >2000 | 73 |
| 40 | H | i-propyl* | H | 3-CH₃ | -4-CH₃ | 0.26 | >2000 | 121 |
| 41 | H | i-propyl | H | H | 4-t-butyl | 3.5 | >2000 | 75 |
| 42 | H | ethyl | 3-F | H | H | 4.25 | >2000 | 136 |
| 43 | H | methyl | 3-F | H | H | 22 | >2000 | 540 |

| | | | | | | | | |
|---|---|---|---|---|---|---|---|---|
| * indicates "S" stereochemistry | | | | | | | | |
| ** indicates "R" stereochemistry | | | | | | | | |

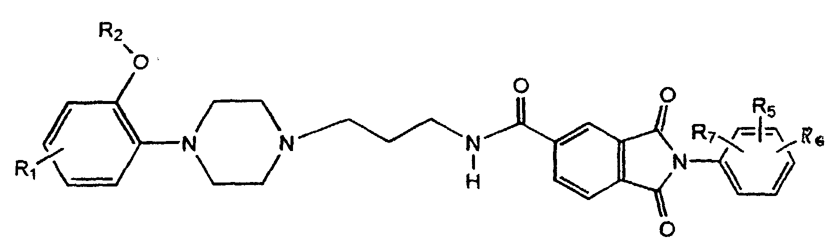

**TABLE B**

| RWJ | R₁ | R₂ | R₅ | P₆ | R₇ | α1a | α1b | α1d |
|---|---|---|---|---|---|---|---|---|
| 6 | H | i-propyl | 3-F | H | H | 0.41 | 482 | 22 |
| 44 | H | i-propyl | H | H | 4-N(CH₃)₂ | 0.18 | 465 | 15 |
| 45 | H | i-propyl | H | H | 4-OCH₃ | 0.11 | 361 | 16 |
| 46 | H | i-propyl | H | H | 4-CH₃ | 0.1 | >2000 | 37 |
| 47 | H | i-propyl | H | H | 4-OH | 0.2 | 255 | 13 |

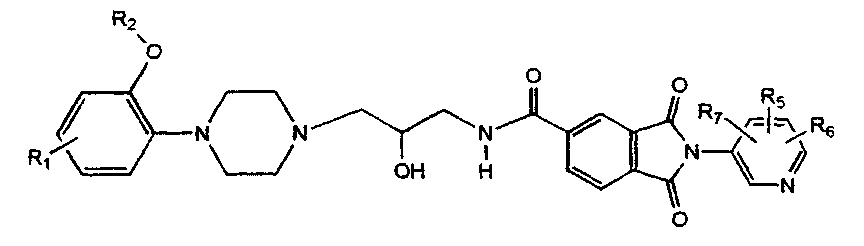

**TABLE C**

| Cpd. | R₁ | R₂ | R₅ | R₆ | R₇ | α1a | α1b | α1d |
|---|---|---|---|---|---|---|---|---|
| 11 | H | i-propyl | H | H | H | 1.9 | >2000 | 31 |

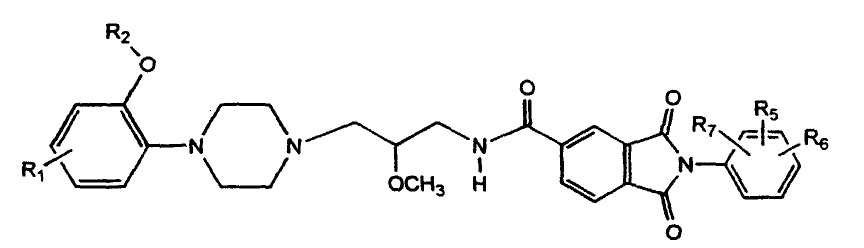

**TABLE D**

| Cpd. | R₁ | R₂ | R₅ | R₆ | R₇ | α1a | α1b | α1d |
|---|---|---|---|---|---|---|---|---|
| 14 | H | i-propyl | 3-F | H | H | 7 | >2000 | 407 |

### Example 23

The selectivity of the compounds of the invention for prostate tissues over aortic tissues was demonstrated as follows. The contractile responses of rat prostatic tissue and rat aorta tissues were examinea in the presence and absence of antagonist compounds. As an indication of the selectivity of antagonism, test compound effects on vascular smooth muscle contractility (α1_{b}-AR and α1_{d}-AR) were compared to the effects on prostatic smooth muscle (α1ₐ-AR). Strips of prostatic tissue and aortic rings were obtained from Long Evans derived male rats weighing 275 grams and sacrificed by cervical dislocation. The prostate tissue was placed under 1 gram tension in a 10 ml bath containing phosphate buffered saline pH 7.4 at 32 °C. and isometric tension was measured with force transducers. The aortic tissue was placed under 2 grams tension in a 10 ml bath containing phosphate buffered saline pH 7.4 at 37 °C. The ability of test compound to reduce the norepinephrine-induced contractile response by 50 % (IC₅₀) was determined. Compound 4 inhibited the contractile response in aortic tissue with an IC₅₀ of 63.2 µM and in prostate tissue with an IC₅₀ of 0.64 µM. Compound 6 inhibited the contractile response in aortic tissue with an IC₅₀ of 2.8 µM and in prostate tissue with an IC₅₀ of 0.13 µM. Compound 9 inhibited the contractile response in aortic tissue with an IC₅₀ of 6.5 µM and in prostate tissue with an IC₅₀ of 0.23 µM. Compound 45 inhibited the contractile response in aortic tissue with an IC₅₀ of 3.3 µM and in prostate tissue with an IC₅₀ of 0.04 µM. Compound 34 inhibited the contractile response in aortic tissue with an IC₅₀ of 42.5 µM and in prostate tissue with an IC₅₀ of 0.75 µM. Compound 21 inhibited the contractile response in aortic tissue with an IC₅₀ of 22.4 µM and in prostate tissue with an IC₅₀ of 0.81 µM.

### Example 24

Select compounds of the invention were tested for their ability to antagonize phenylephrine (PE) induced increases in intraurethral pressure in dogs. The selectivity of these compounds was demonstrated by comparing their effect upon PE induced increases in mean arterial pressure (MAP) in the dog.

Male beagle dogs were anesthetized and catheterized to measure intraurethral pressure (IUP) in the prostatic urethra. Mean arterial pressure (MAP) was measured using a catheter placed in the femoral artery. Dogs were initially administered six i.v. bolus doses (1 to ≤ 32mg/kg) of phenylephrine (PE) to establish a control agonist dose-response curve. IUP and MAP were recorded following each dose until the IUP returned to baseline. The does then were given an i.v. bolus dose of the antagonist compound, followed by i.v. PE challenges of ascending doses, as in the control agonist dose-response curve. IUP and MAP measurements following each PE challenge were recorded. The antagonist compound was tested over a dose range of 3 to 300 ug/kg in half-log increments. The interval between antagonist doses was at least 45 min and three experiments were performed for each test compound. The graphs below illustrates the mean percentage reductions in IUP and MAP for compounds 21 and 46 respectively.

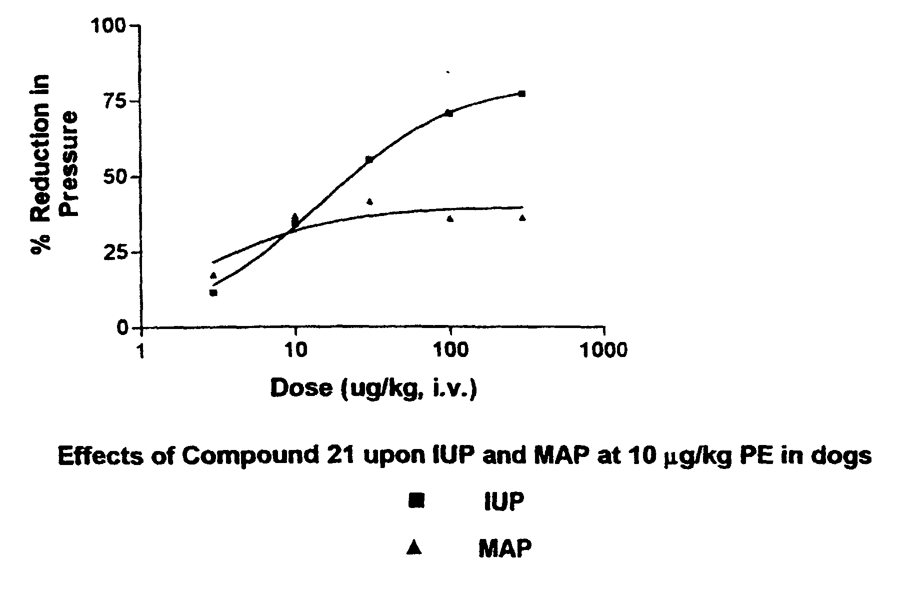

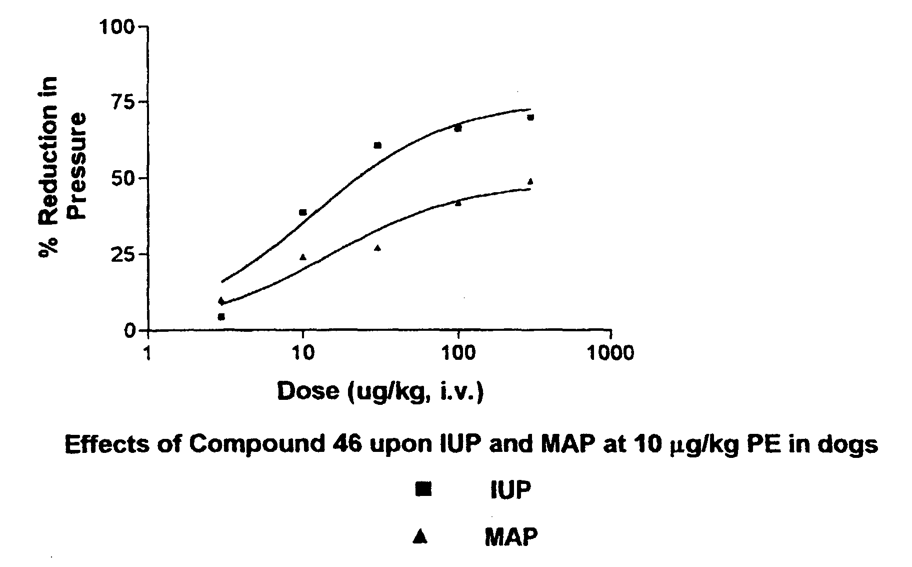

### Example 25

The duration of select compounds of the invention was determined by the measuring the IUP and MAP responses to repeated PE challenges in conscious dogs over time. Male beagles dogs were instrumented for the continuous measurement of arterial blood pressure by implanting a catheter containing a pressure transducer into the abdominal aorta via the femoral artery. The telemetry transmitter was positioned subcutaneously in the animal's flank. IUP was monitored with a catheter positioned in the prostatic urethra. Prior to antagonist test compound administration, the IUP and MAP responses to a 20 µg/kg i.v. dose of PE were determined and repeated several times to establish a baseline (100% maximal) response. An oral bolus dose of antagonist was administered, followed by a 20 µg/kg i.v. PE challenge at 0.5, 1, 1.5, 2, 4, 6, 12, and 24 hours post dosing. The IUP and MAP responses following each PE challenge were recorded. Compound 33 was tested at doses of 0.1, 0.3, and 1 mg/kg. The IUP and MAP responses at each time point following the PE challenge are presented in the following graphs as the percent of the maximal response. 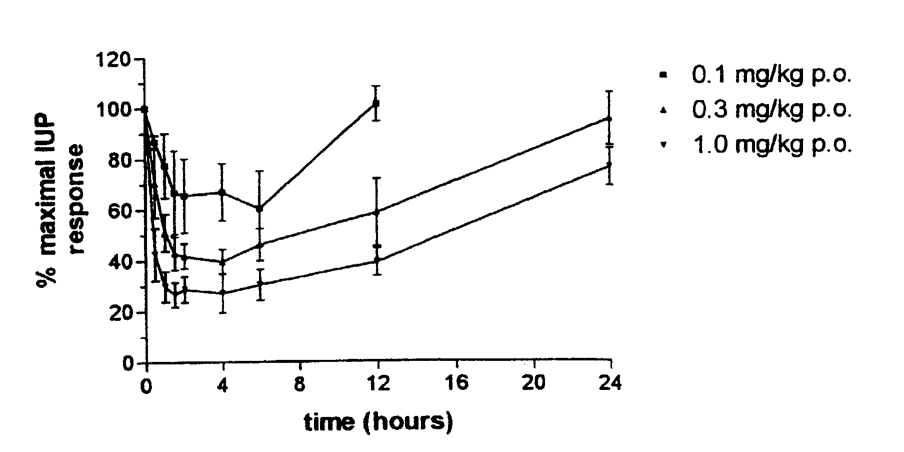 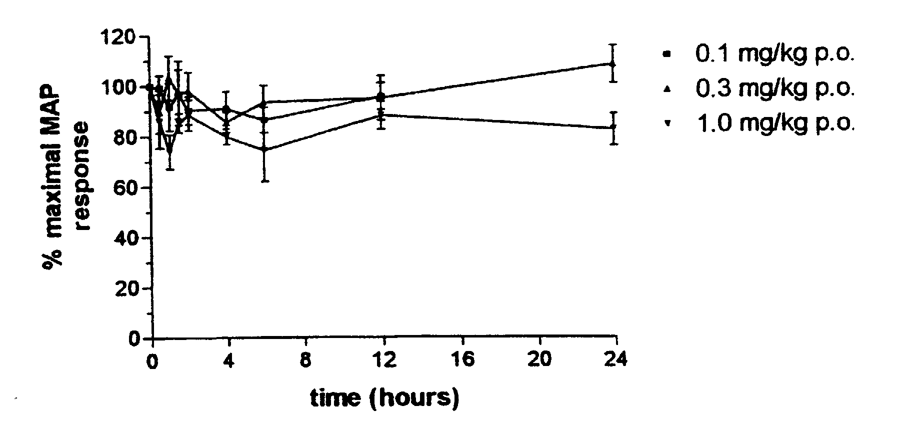 **Duration Studies Compound 21**

### REFERENCES

M. Barry & C. Roehbom, Management of Benign Prostatic Hyperplasia, 48 Annu. Rev. Med. 177-89 (1997),
Bruno JF, Whittaker J, Song J, and Berelowitz M. (1991) Molecular cloning and sequencing of a cDNA encoding a human α1A adrenergic receptor. Biochem. Biophys. Res. Commun. 179: 1485-1490.
Forray C, Bard JA, Wetzel JM, Chiu G, Shapiro E, Tang R, Lepor H, Hartig PR, Weinshank RL, Branchek TA, and Gluchowski C (1994) The α1-adrenergic receptor that mediates smooth muscle contraction in human prostate has the pharmacological properties of the cloned human α1c subtype. Mol. Pharmacol. 45: 703-708.
Gormley G, Stoner E, Bruskewitz RC et al. (1992) The effect of finasteride in men with benign prostatic hyperplasia. N. Engl. J. Med. 327: 1185-1191.
Hatano A, Takahashi H, Tamaki M, Komeyama T, Koizumi T, and Takeda M (1994) Pharmacological evidence of distinct α1-adrenoceptor subtypes mediating the contraction of human prostatic urethra and peripheral artery. Br. J. Pharmacol. 113: 723-728.
Harrison JK, Pearson WR, and Lynch KR (1991) Molecular characterization of α1- and α2-adrenoceptors. Trends Pharmacol. Sci. 12: 62-67.
Hieble JP and Caine M (1986) Etiology of benign prostatic hyperplasia and approaches to pharmacological management. Fed. Proc. 45: 2601 - 2603.
Hirasawa A, Horie K, Tanaka T, Takagaki K, Murai M, Yano J, and Tsujimoto G (1993) Cloning, functional expression and tissue distribution of human cDNA for the α1c-adrenergic receptor. Biochem. Biophys. Res. Commun. 195: 902-909.
Lepor H and Rigaud G (1990) The efficacy of transurethral resection of the prostate in men with moderate symptoms of prostatism. J. Urol. 143: 533-537.
Lepor H, Auerbach S, Puras-Baez A et al. (1992) A randomized, placebo-controlled multicenter study of the efficacy and safety of terazosin in the treatment of benign prostatic hyperplasia. J. Urol. 148: 1467-1474
Lepor H (1995) α-Blockade for benign prostatic hyperplasia (BPH) Clin. Endocrinol. Metab. 80: 750-753.
Marshall I, Burt RP, Andersson PO, Chapple CR, Greengrass PM, Johnson GI, and Wyllie MG (1992) Human α1c-adrenoceptor: functional characterisation in prostate. Br. J. Pharmacol. 107(Proc. Suppl. Dec.):327P.
Marshall I, Burt RP, and Chapple CR (1995) Noradrenaline contractions of human prostate mediated by α1A- (α1c-) adrenoceptor subtype. Br. J. Pharmacol. 115: 781-786.
Mebust WK, Holtgrewe HL, Cockett ATK, and Peters PC (1989) Transurethral prostatectomy: immediate and postoperative complications. A cooperative study of 13 participating institutions evaluating 3,885 patients.J. Urol. ,141: 243-247.
Oesterling JE (1995) Benign prostatic hyperplasia. Medical and minimally invasive treatment options. N. Engl. J. Med. 332: 99-109.
Ramarao CS, Kincade Denker JM, Perez DM, Gaivin RJ, Riek RP, and Graham RM (1992) Genomic organization and expression of the human α1B-adrenergic receptor. J. Biol. Chem. 267: 21936-21945.
Schwinn DA, Johnston GI, Page SO, Mosley MJ, Wilson KH, Worman NP, Campbell S, Fidock MD, Fumess LM, Parry-Smith DJ, Peter B, and Bailey DS (1995) Cloning and pharmacological characterization of human alpha-1 adrenergic receptors: sequence corrections and direct comparison with other species homologues. JPET 272: 134-142.
William D. Steers & Burkhart Zorn, Benign Prostatic Hyperplasia, in Disease-a-Month (M. Greenbeerger et al. Eds., 1995).
Weinberg DH, Trivedi P, Tan CP, Mitra S, Perkins-Barrow A, Borkowski D, Strader CD, and Bayne M (1994) Cloning, expression and characterization of human α adrenergic receptors α1A, α1B, and α1C. Biochem. Biophys. Res. Commun. 201: 1296-1304.
Weis KA, Epstein RS, Huse DM, Deverka PA and Oster G (1993) The costs of prostatectomy for benign prostatic hyperplasia. Prostate 22: 325-334.
Wennberg JE, Roos N, Sola L, Schori A, and Jaffe R (1987) Use of claims data systems to evaluate health care outcomes: mortality and reoperation following prostatectomy. JAMA 257: 933-936.
Yamada S, Tanaka C, Kimura R, and Kawabe K (1994) Alpha 1-adrenoceptors in human prostate: characterization and binding characteristics of alpha 1-antagonists. Life Sci. 54: 1845-1854.

## Claims

1. A compound of Formula I 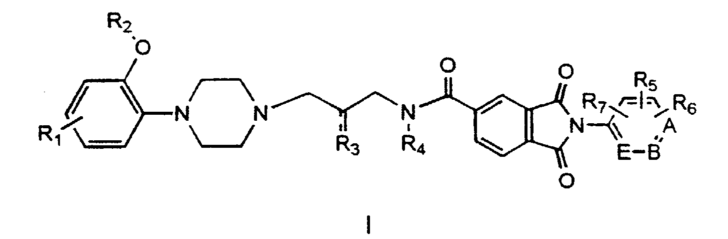 wherein:
R₁ is hydrogen, halogen, C₁₋₅alkoxy, hydroxyl, or C₁₋₅alkyl;
R₂ is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
R₃ is hydrogen, hydroxy or C₁₋₅alkoxy if the hashed line is absent or
is oxygen if the hashed line is present;
R₄ is hydrogen, C₁₋₅alkyl, phenylC₁₋₅alkyl or substituted phenylC₁₋₅alkyl
where the phenyl substitutents are independently selected from one or more members of the group consisting of C₁₋₅alkyl, C₁₋₅alkoxy, and trihaloC₁₋₅alkyl;
R₅ is hydrogen, halogen, hydroxy, C₁₋₈alkyl, substituted C₁₋₈alkyl
where the alkyl substitutents are independently selected from one or more halogens,
C₁₋₅alkoxy, amino, C₁₋₅alkylamino, diC₁₋₅alkylamino, C₁₋₅alkylcarbonyl,
C₁₋₅alkoxycarbonyl, nitrile, or nitro;
R₆ is hydrogen, halogen, hydroxy, C₁₋₈alkyl, substituted C₁₋₈alkyl
where the alkyl substitutents are independently selected from one or more halogens,
C₁₋₅alkoxy, amino, C₁₋₅alkylamino, diC₁₋₅alkylamino, C₁₋₅alkylcarbonyl,
C₁₋₅alkoxycarbonyl, or nitro;
R₇ is hydrogen, halogen, hydroxy, C₁₋₈alkyl, substituted C₁₋₈alkyl
where the alkyl substitutents are independently selected from one or more halogens,
C₁₋₅alkoxy, amino, C₁₋₅alkylamino, diC₁₋₅alkylamino, C₁₋₅alkylcarbonyl,
C₁₋₅alkoxycarbonyl, or nitro;
A is nitrogen or carbon;
B is nitrogen or carbon;
E is nitrogen or carbon;
with the proviso that only one of A, B, or E is nitrogen;
pharmaceutically acceptable salts thereof; and
stereoisomers, racemic mixtures, as well as enantiomers thereof.

2. The compound of claim 1 wherein
R₁ is hydrogen, halogen or hydroxy,
R₂ is C₁₋₆alkyl, phenyl, substituted phenyl, phenyl C₁₋₆alkyl or hydrogen,
R₃ is hydroxy or hydrogen,
R₄ is hydrogen or C₁₋₅alkyl,
R₅ is hydrogen, halogen, hydroxy, C₁₋₈alkyl, C₁₋₅alkoxy, amino, diC₁₋₅alkylamino, C₁₋₅alkylcarbonyl, or nitrile,
R₆ is hydrogen, halogen,hydroxy, C₁₋₈alkyl, C₁₋₅alkoxy, amino, diC₁₋₅alkylamino, C₁₋₅alkylcarbonyl, or nitrile,
R₇ is hydrogen, halogen,hydroxy, C₁₋₈alkyl, C₁₋₅alkoxy, amino, diC₁₋₅alkylamino, C₁₋₅alkylcarbonyl, or nitrile, and
A, B, and E are carbon.

3. The compound of claim 1 wherein
R₁ is hydrogen,
R₂ is C₁₋₆alkyl, phenyl or substituted phenyl,
R₃ is hydroxy or hydrogen,
R₄ is hydrogen,
R₅ is hydrogen, halogen, hydroxy, C₁₋₈alkyl, C₁₋₅alkoxy,
C₁₋₅alkylcarbonyl, or diC₁₋₅alkylamino,
R₆ is hydrogen, halogen, hydroxy, C₁₋₈alkyl, C₁₋₅alkoxy, C₁₋₅alkylcarbonyl, or diC₁₋₅alkylamino,
R₇ is hydrogen, halogen, hydroxy, C₁₋₈alkyl, C₁₋₅alkoxy, C₁₋₅alkylcarbonyl, or diC₁₋₅alkylamino, and
A, B, and E are carbon.

4. The compound of claim 1 wherein
R₁ is hydrogen,
R₂ is C₁₋₆alkyl, substituted phenyl,
R₃ is hydroxy,
R₄ is hydrogen,
R₅ is hydrogen, halogen, hydroxy, C₁₋₈alkyl, C₁₋₅alkoxy, C₁₋₅alkylcarbonyl, or diC₁₋₅alkylamino,
R₆ is hydrogen, halogen, hydroxy, C₁₋₈alkyl, C₁₋₅alkoxy, C₁₋₅alkylcarbonyl, or diC₁₋₅alkylamino,
R₇ is hydrogen, halogen, hydroxy, C₁₋₈alkyl, C₁₋₅alkoxy, C₁₋₅alkylcarbonyl, or diC₁₋₅alkylamino, and
A, B, and E are carbon.

5. The compound of claim 1 wherein R₁ is hydrogen, R₂ is isopropyl, R₃ is hydroxy, R₄ is hydrogen and R₆ and R₇ are hydrogen and R₅ is either 4-dimethylamino or 4-methyl.

6. The compound of claim 1 wherein R₃ is hydroxy and R₅ is amino, C₁₋₅alkylamino or diC₁₋₅alkylamino.

7. The compound of claim 1 selected from the group consisting of
2-[4-(fluoro)phenyl]-2,3-dihydro-N-[2-hydroxy-3-[4-[2-(1-methylethoxy)phenyl]-1-piperazinyl]propyl]-1,3-dioxo-1H-isoindole-5-carboxamide;
2-[3-fluorophenyl]-2,3-dihydro-N-[2-hydroxy-3-[4-[2-(1-methylethoxy)phenyl]-1-piperazinyl]propyl]-1,3-dioxo-1 H-isoindole-5-carboxamide;
2-[4-(dimethylamino)phenyl]-2,3-dihydro-N-[2-hydroxy-3-[4-[2-(1-methylethoxy)phenyl]-1 -piperazinyl]propyl]-1,3-dioxo-1 H-isoindole-5-carboxamide;
2-[4-methylphenyl]-2,3-dihydro-N-[2-hydroxy-3-[4-[2-(1-methylethoxy)phenyl]-1-piperazinyl]propyl]-1,3-dioxo-1H-isoindole-5-carboxamide;
2-[3,4,5-trimethoxyphenyl]-2,3-dihydro-N-[2-hydroxy-3-[4-[2-methylethoxy)phenyl]-1-piperazinyl]propyl]-1,3-dioxo-1H-isoindole-5-carboxamide;
2-[4-chlorophenyl]-2,3-dihydro-N-[2-hydroxy-3-[4-[2-(1-methylethoxy)phenyl]-1-piperazinyl]propyl]-1,3-dioxo-1 H-isoindole-5-carboxamide;
2-[4-hydroxyphenyl]-2,3-dihydro-N-[2-hydroxy-3-[4-[2-(1-methylethoxy)phenyl]-1-piperazinyl]propyl]-1,3-dioxo-1H-isoindole-5-carboxamide;
2-[5-methoxyphenyl]-2, 3-dihydro-N-[2-hydroxy-3-[4-[2-(1-methylethoxy)phenyl]-1-piperazinyl]propyl]-1,3-dioxo-1H-isoindole-5-carboxamide;
2-[4-ethylphenyl]-2,3-dihydro-N-[2-hydroxy-3-[4-[2-(1-methylethoxy)phenyl]-1-piperazinyl]propyl]-1,3-dioxo-1 H-isoindole-5-carboxamide;
(S)-2-[4-(dimethylamino)phenyl]-2,3-dihydro-N-[2-hydroxy-3-[4-[2-(1-methylethoxy)phenyl]-1-piperazinyl]propyl]1,3-dioxo-1H-isoindole-5-carboxamide;
2-[4-(fluoro)phenyl]-2,3-dihydro-N-[3-[4-[2-( 1-methy(ethoxy)phenyl]-1-piperazinyl]propyl]-1,3-dioxo-1 H-isoindole-5-carboxamide;
2-[3-fluorophenyl]-2, 3-dihydro-N-[3-[4-[2-(1-methylethoxy)phenyl]-1-piperazinyl]propyl]-1,3-dioxo-1 H-isoindole-5-carboxamide;
2-[4-(dimethylamino)phenyl]-2, 3-dihydro-N-[3-[4-[2-(1-methylethoxy)phenyl]-1-piperazinyl]propyl]-1,3-dioxo-1 H-isoindole-5-carboxamide;
2-[4-methylphenyl]-2,3-dihydro-N-[3-[4-[2-(1-methylethoxy)phenyl]-1-piperazinyl]propyl]-1,3-dioxo-1 H-isoindole-5-carboxamide;
2-[3,4,5-trimethoxyphenyl]-2,3-dihydro-N-[3-[4-[2-(1-methylethoxy)phenyl]-1 -piperazinyl]propyl]-1,3-dioxo-1H-isoindole-5-carboxamide;
2-[4-chlorophenyl]-2,3-dihydro-N-[3-[4-[2-(1-methylethoxy)phenyl]-1-piperazinyl]propyl]-1,3-dioxo-1 H-isoindole-5-carboxamide;
2-[4-hydroxyphenyl]-2,3-dihydro-N-[3-[4-[2-(1-methylethoxy)phenyl]-1-piperazinyl]propyl]-1,3-dioxo-1 H-isoindole-5-carboxamide;
2-[5-methoxyphenyl]-2,3-dihydro-N-[3-[4-[2-(1-methylethoxy)phenyl]-1-piperazinyl]propyl]-1,3-dioxo-1 H-isoindole-5-carboxamide; and
2-[4-ethylphenyl]-2,3-dihydro-N-[3-[4-[2-(1-methylethoxy)phenyl]-1-piperazinyl]propyl]-1,3-dioxo-1H-isoindole-5-carboxamide.

8. A pharmaceutical composition containing an effective dose of a compound of Formula 1 as defined in any one of claims 1 to 7.

9. The pharmaceutical composition of claim 8 wherein the effective dose of a compound of Formula 1 is 0.01 to 25.0 mg/kg, preferably 0.1 to 1.0 mg/kg.

10. The compound of any one of claims 1 to 7 or the composition of claim 8 or claim 9 for use in treating either benign prostatic hyperplasia or diseases associated with the α 1a adrenergic receptor.

11. A compound of Formula II 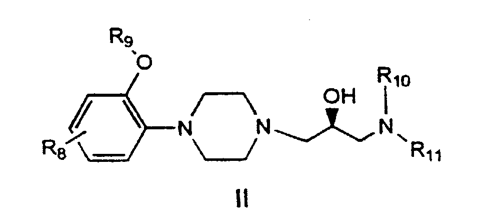 wherein
R₈ is hydrogen, halogen, C₁₋₅alkoxy, hydroxyl, or C₁₋₅alkyl;
R₉ is C₁₋₆alkyl, substituted C₁₋₆alkyl
where the alkyl substituents are independently selected from one or more halogens,
phenyl,
substituted phenyl
where the phenyl substituents are independently selected from one or more members of the group consisting of C₁₋₅alkyl, C₁₋₅alkoxy, and trihaloC₁₋₅alkyl,
phenylC₁₋₅alkyl, or
substituted phenylC₁₋₅alkyl
where the phenyl substituents are independently selected from one or more members of the group consisting of C₁₋₅alkyl, halogen, C₁₋₅alkoxy, and trihaloC₁₋₅alkyl;
R₁₀ is hydrogen, C₁₋₅alkoxycarbonyl, phenylC₁₋₅alkoxycarbonyl, or allyloxycarbonyl;
R₁₁ is hydrogen, phenylC₁₋₅alkyl, or substituted phenylC₁₋₅alkyl;
where the phenyl substituents are independently selected from one or more members of the group consisting of C₁₋₅alkyl, halogen, C₁₋₅alkoxy, and nitro;

12. The compound of claim 11 where R₈ is hydrogen or halogen, R₉ is C₁₋₆alkyl, phenyl or substituted phenyl, R₁₀ is hydrogen or C₁₋₅alkoxycarbonyl, and R₁₁ is hydrogen, phenylC₁₋₅alkyl or C₁₋₅alkoxy substituted phenylC₁₋₅alkyl.

13. The compound of claim 12 wherein R₈ is hydrogen, R₉ is isopropyl, R₁₀ is C₁₋₅alkoxycarbonyl, and R₁₁ is phenylC₁₋₅alkyl.

14. The compound of claim 13 wherein R₈ is hydrogen, R₉ is isopropyl, R₁₀ is t-butoxycarbonyl, and R₁₁ is benzyl.

15. The compound of claim 12 wherein R₈ is hydrogen, R₉ is isopropyl, R₁₀ is hydrogen, and R₁₁ is benzyl.

16. The compound of claim 12 wherein R₈ is hydrogen, R₉ is isopropyl, R₁₀ is hydrogen, and R₁₁ is hydrogen

17. A compound of Formula III 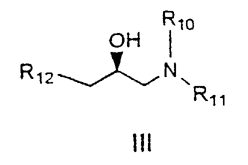 wherein
R₁₀ is C₁₋₅alkoxycarbonyl, phenylC₁₋₅alkoxycarbonyl, or allyloxycarbonyl;
R₁₁ is phenylC₁₋₅alkyl, or substituted phenylC₁₋₅alkyl
where the phenyl substituents are independently selected from one or more members of the group consisting of C₁₋₅alkyl, halogen, C₁₋₅alkoxy, and nitro;
R₁₂ is halogen, hydroxyl, mesyl, or tosyl.

18. The compound of claim 17 wherein R₁₀ is t-butoxycarbonyl and R₁₁ is benzyl.

19. The compound of claim 17 or claim 18 wherein R₁₂ is chloro.

20. Reacting a compound of Formula 111 as defined in any one of claims 17 to 19, provided that R₁₂ is not hydroxyl, with a piperazine derivative of Formula IV 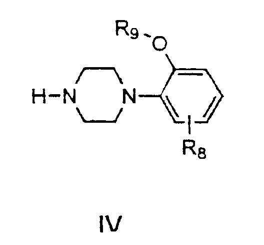 wherein R₈ and R₉ are as defined in any one of claims 11 to 16, in the presence of a basic reagent to produce a compound for Formula 11 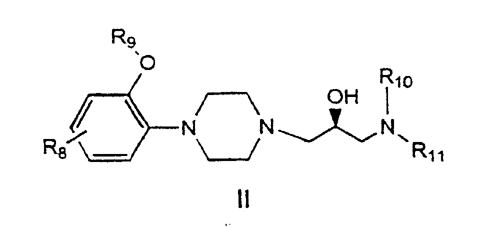 wherein R₈, R₉, R₁₀ and R₁₁ are as defined earlier in this claim.

21. The method of claim 20 where the basic reagent is potassium hydroxide.

22. Reacting a compound of Formula II 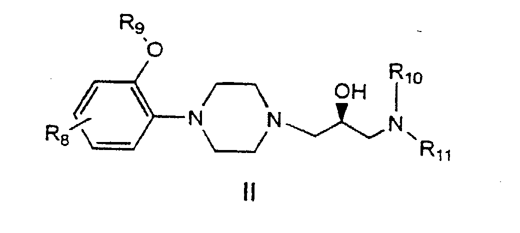 wherein R₈, R₉, R₁₀ and R₁₁ are as defined in any one of claims 11 to 16, provided that neither R₁₀ nor R₁₁ is hydrogen, with an acidic reagent to give a compound of Formula 11. 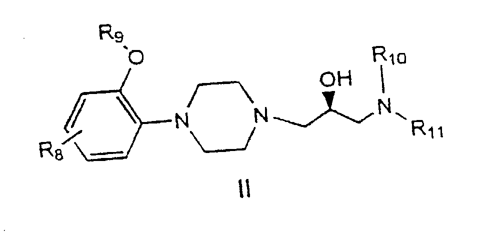 wherein R₈, R₉ and R₁₁ are as defined earlier in this claim and R₁₀ is hydrogen.

23. The method of claim 22 where the acidic reagent is trifluoroacetic acid.

24. Reacting a compound of Formula II 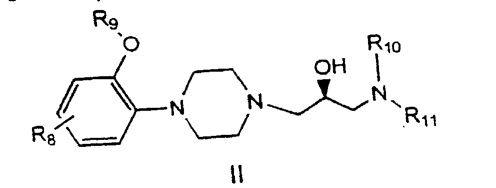 wherein R₈, R₉, R₁₁ are as defined in any one of claims 11 to 16, provided that R₁₁ is not hydrogen, and
with a reducing agent to give a compound of Formula II 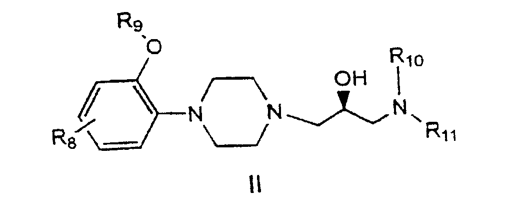 wherein R₈, R₉ and R₁₀ are as defined earlier in this claim and R₁₁ is hydrogen.

25. The method of claim 24 wherein the reducing agent is ammonium formate and Pd/C.
